# EUROPEAN PATENT APPLICATION

(11) **EP 4 763 858 A1**
(43) Date of publication of application: **24.06.2026**
(21) Application number: 24881516.9
(22) Date of filing: 18.10.2024
(51) Int. Cl.: C07K 14/725, C07K 7/06, A61P 35/00

(54) **T-CELL RECEPTOR, AND PREPARATION METHOD THEREFOR AND USE THEREOF**

(30) Priority: 26.10.2023 CN 202311401733; 09.10.2024 CN 202411403067
(71) Applicant: Shanghai General Hospital, Shanghai 200080 (CN)
(72) Inventor: SHEN, Lianghua, Shanghai 200080 (CN); WANG, Pengran, Shanghai 200080 (CN); DING, Xiaodan, Shanghai 200080 (CN); XU, Jian, Shanghai 200080 (CN); MA, Ling, Shanghai 200080 (CN); ZHANG, Yan, Shanghai 200080 (CN); SONG, Xianmin, Shanghai 200080 (CN)
(74) Representative: Canzler & Bergmeier Patentanwälte Partnerschaft mbB
(86) International application number: PCT/CN2024/125613
(87) International publication number: WO 2025/087151

(57) **Abstract**

The present invention relates to the field of biomedicine. Provided are a T-cell receptor, and a preparation method therefor and the use thereof. The T-cell receptor EBV-TCR comprises a TCRα chain variable region and a TCRβ chain variable region; the TCRα chain variable region comprises peptide fragments CDRα1, CDRα2 and CDRα3; the TCRβ chain variable region comprises peptide fragments CDRβ1, CDRβ2 and CDRβ3; and the amino acid sequence of the CDRα3 is as set forth in SEQ ID NO. 7, and the amino acid sequence of the CDRβ3 is as set forth in SEQ ID NO. 3. The TCR has a high specificity and can significantly activate the release of cytokines when binding to T-cells, thus providing a theoretical and experimental basis for developing a TCR-T therapy targeting an EB virus antigen.

## Description

### FIELD OF TECHNOLOGY

The present disclosure relates to the technical field of biomedicine, and in particular to a T cell receptor, a preparation method and use thereof.

### BACKGROUND

Epstein-Barr virus (EBV) is a common double-stranded DNA herpesvirus. Approximately 90% of the global population has been infected with EBV (Tzellos, S.; Farrell, P. J. Epstein-Barr virus sequence variation-biology and disease. Pathogens, 2012, 1(2): 156-174). Studies have demonstrated that EBV infection is closely associated with the occurrence of various diseases, including post-hematopoietic stem cell transplantation (HSCT) infection, lymphoma, and nasopharyngeal carcinoma (Taylor, G. S., Long, H. M., Brooks, J. M., Rickinson, A. B., Hislop, A. D. The immunology of Epstein-Barr virus-induced disease. Annu Rev Immunol, 2015, 33: 787-821). In addition, approximately 1-2% of all human malignancies are attributable to EBV infection, resulting in about 200,000 new cancer cases annually worldwide (Münz, C. Latency and lytic replication in Epstein-Barr virus-associated oncogenesis. Nat Rev Microbiol, 2019, 17(11): 691-700). Therefore, therapeutic strategies targeting EBV are of significant importance.

The EBV infection cycle mainly includes a lytic phase and a latent phase, during which different viral proteins are expressed. Proteins expressed during the lytic phase as well as those expressed during the latent phase (primarily including six nuclear proteins EBNA1, EBNA2, EBNA3A, EBNA3B, EBNA3C, EBNA-LP, and three latent membrane proteins LMP1, LMP2A, and LMP2B, wherein LMP2B is a truncated isoform of LMP2A) are considered to play important roles in tumorigenesis. Moreover, EBV-associated tumor cells express EBV antigenic proteins, and T cell-mediated cytotoxicity constitutes the principal mechanism by which the human body combats viral infection. Accordingly, EBV antigenic proteins represent ideal targets for T cell-mediated immunotherapy.

T cell receptor engineered T cell therapy (TCR-T therapy) is an adoptive T cell therapy that has rapidly developed in recent years. Unlike chimeric antigen receptor T cell therapy (CAR-T therapy), which can only recognize cell-surface antigens, TCR-T therapy is capable of recognizing both intracellular and extracellular targets through the human leukocyte antigen (HLA) antigen presentation system. In addition, TCR-T cells exhibit higher sensitivity; theoretically, a single antigen molecule on a target cell is sufficient to activate TCR-T cells and elicit cytotoxic activity. TCR-T therapy has therefore demonstrated great potential in the treatment of both hematological malignancies and solid tumors.

Most previously disclosed inventions have reported TCRs targeting only a single EBV antigen peptide or a single HLA subtype, and have not provided complete functional validation data for the TCRs (including CN101182531A, CN106632659A, CN107001444B, and CN111690050A). Although CN108602875A and CN109306005A provide complete TCR functional validation data, they disclose TCRs targeting only a single EBV antigen peptide or a single HLA subtype.

### SUMMARY

The present disclosure provides a T cell receptor (TCR), as well as a preparation method and use thereof.

The present disclosure provides a T cell receptor, wherein the T cell receptor includes a T cell receptor BMLF1-TCR, a T cell receptor LMP1-TCR1, a T cell receptor LMP1-TCR2, a T cell receptor EBNA-3B-TCR, or a T cell receptor LMP2-TCR, wherein:
I) the T cell receptor BMLF1-TCR includes a TCR α-chain variable region and a TCR β-chain variable region; wherein the TCR α-chain variable region of the T cell receptor BMLF1-TCR includes CDR α1, CDR α2, and CDR α3, and the TCR β-chain variable region of the T cell receptor BMLF1-TCR includes CDR β1, CDR β2, and CDR β3; wherein an amino acid sequence of CDR α3 of the T cell receptor BMLF1-TCR is as shown in SEQ ID NO. 7, and an amino acid sequence of CDR β3 of the T cell receptor BMLF1-TCR is as shown in SEQ ID NO. 3;
II) a TCR α-chain variable region of the T cell receptor LMP1-TCR1 includes CDR α1, CDR α2, and CDR α3, and a TCR β-chain variable region of the T cell receptor LMP1-TCR1 includes CDR β1, CDR β2, and CDR β3; wherein an amino acid sequence of CDR α3 of the T cell receptor LMP1-TCR1 is as shown in SEQ ID NO. 15, and an amino acid sequence of CDR β3 of the T cell receptor LMP1-TCR1 is as shown in SEQ ID NO. 11;
III) a TCR α-chain variable region of the T cell receptor LMP1-TCR2 includes CDR α1, CDR α2, and CDR α3, and a TCR β-chain variable region of the T cell receptor LMP1-TCR2 includes CDR β1, CDR β2, and CDR β3; wherein an amino acid sequence of CDR α3 of the T cell receptor LMP1-TCR2 is as shown in SEQ ID NO. 23, and an amino acid sequence of CDR β3 of the T cell receptor LMP1-TCR2 is as shown in SEQ ID NO. 19;
IV) a TCR α-chain variable region of the T cell receptor EBNA-3B-TCR includes CDR α1, CDR α2, and CDR α3, and a TCR β-chain variable region of the T cell receptor EBNA-3B-TCR includes CDR β1, CDR β2, and CDR β3; wherein an amino acid sequence of CDR α3 of the T cell receptor EBNA-3B-TCR is as shown in SEQ ID NO. 33, and an amino acid sequence of CDR β3 of the T cell receptor EBNA-3B-TCR is as shown in SEQ ID NO. 29;
V) a TCR α-chain variable region of the T cell receptor LMP2-TCR includes CDR α1, CDR α2, and CDR α3, and a TCR β-chain variable region of the T cell receptor LMP2-TCR includes CDR β1, CDR β2, and CDR β3; wherein an amino acid sequence of CDR α3 of the T cell receptor LMP2-TCR is as shown in SEQ ID NO. 42, and an amino acid sequence of CDR β3 of the T cell receptor LMP2-TCR is as shown in SEQ ID NO. 38.

The present disclosure further provides an EBV antigen peptide, wherein the EBV antigen peptide includes an antigen peptide BMLF1, an antigen peptide LMP1, an antigen peptide EBNA3, or an antigen peptide LMP2, wherein:
the amino acid sequence of the antigen peptide BMLF1 is as shown in SEQ ID NO. 25; or
the amino acid sequence of the antigen peptide LMP1 is as shown in SEQ ID NO. 26; or
the amino acid sequence of the antigen peptide EBNA3 is as shown in SEQ ID NO. 35; or
the amino acid sequence of the antigen peptide LMP2 is as shown in SEQ ID NO. 44.

The present disclosure further provides a method for preparing the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, or the aforementioned T cell receptor LMP2-TCR, wherein the preparation method includes the following steps:
1) loading the aforementioned EBV antigen peptide onto *in vitro*-differentiated dendritic cells (DCs); and
2) co-culturing the dendritic cells obtained in step 1) with CD8+ T cells, thereby obtaining the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, or the aforementioned T cell receptor LMP2-TCR.

The present disclosure further provides a biological material, wherein the biological material includes a polynucleotide, a nucleic acid construct, a virus, or a cell;
wherein the polynucleotide encodes the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, or the aforementioned T cell receptor LMP2-TCR; or the polynucleotide encodes the aforementioned EBV antigen peptide;
the nucleic acid construct includes the aforementioned polynucleotide and a plasmid backbone;
the virus includes the aforementioned polynucleotide;
the cell includes the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, the aforementioned T cell receptor LMP2-TCR, the aforementioned polynucleotide, the aforementioned nucleic acid construct, or the aforementioned virus.

The present disclosure further provides a use of the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, the aforementioned T cell receptor LMP2-TCR, the aforementioned EBV antigen peptide, or the aforementioned biological material in the preparation of a tumor therapeutic product.

The present disclosure further provides a tumor therapeutic product, wherein the tumor therapeutic product includes the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, the aforementioned T cell receptor LMP2-TCR, the aforementioned EBV antigen peptide, or the aforementioned biological material and a pharmaceutically acceptable auxiliary material.

The present disclosure further provides a use of the aforementioned EBV antigen peptide or the aforementioned biological material in the preparation of the following products:
1) an anti-tumor peptide vaccine product; and
2) a product for generating the T cell receptor *in vitro.*

The present disclosure further provides a method for treating a tumor, wherein the method includes administering the aforementioned T cell receptor BMLF1-TCR, the aforementioned T cell receptor LMP1-TCR1, the aforementioned T cell receptor LMP1-TCR2, the aforementioned T cell receptor EBNA-3B-TCR, the aforementioned T cell receptor LMP2-TCR, the aforementioned polynucleotide, the aforementioned nucleic acid construct, the aforementioned virus, or the aforementioned cell to a tumor patient.

As described above, the T cell receptor as well as the preparation method and the use thereof provided by the present disclosure achieve the following beneficial effects:
By taking into account the distribution characteristics of HLA subtypes in the Chinese population, the present disclosure selects four EBV antigen peptides (BMLF1, LMP1, LMP2, and EBNA3B) corresponding to two HLA subtypes (HLA-A*11:01 and HLA-A*02:01) with the highest frequencies in both Chinese and Western populations, systematically screens EBV antigen-specific TCRs, and performs comprehensive functional validation of the TCRs. The T cell receptors of the present disclosure thereby provide a direct basis for the development of TCR-T therapies targeting EBV antigens.

### BRIEF DESCRIPTION OF THE DRAWINGS

FIG. 1 shows a schematic flowchart of the sorting process of TCR-T cells of the present disclosure.
FIG. 2 shows schematic diagrams of the sorting and single-cell sequencing results of the TCR-T cells of the present disclosure.
FIG. 3 shows schematic diagrams of the *in vitro* binding assay results of the TCR-T cells of the present disclosure.
FIG. 4 shows schematic diagrams of the *in vitro* activation assay results of the TCR-T cells of the present disclosure.
FIG. 5 shows schematic diagrams of the *in vitro* cytotoxicity assay results of the TCR-T cells of the present disclosure.
FIG. 6 shows schematic diagrams of the *in vivo* cytotoxicity assay results of some TCR-T cells of the present disclosure.

### DETAILED DESCRIPTION

The present disclosure provides a T cell receptor BMLF1-TCR or a fragment thereof. The T cell receptor BMLF1-TCR includes a TCR α-chain variable region and a TCR β-chain variable region. The TCR α-chain variable region of the T cell receptor BMLF1-TCR includes CDR α1, CDR α2, and CDR α3, and the TCR β-chain variable region of the T cell receptor BMLF1-TCR includes CDR β1, CDR β2, and CDR β3. The amino acid sequence of CDR α3 of the T cell receptor BMLF1-TCR is as shown in SEQ ID NO. 7, and the amino acid sequence of CDR β3 of the T cell receptor BMLF1-TCR is as shown in SEQ ID NO. 3.

In some embodiments, in the TCR α-chain variable region of the T cell receptor BMLF1-TCR, the amino acid sequence of CDR α1 is as shown in SEQ ID NO. 5, or the amino acid sequence of CDR α2 is as shown in SEQ ID NO. 6.

In some embodiments, in the TCR β-chain variable region of the T cell receptor BMLF1-TCR, the amino acid sequence of CDR β1 is as shown in SEQ ID NO. 1, or the amino acid sequence of CDR β2 is as shown in SEQ ID NO. 2.

The present disclosure further provides a T cell receptor LMP1-TCR1 or a fragment thereof. The TCR α-chain variable region of the T cell receptor LMP1-TCR1 includes CDR α1, CDR α2, and CDR α3, and the TCR β-chain variable region of the T cell receptor LMP1-TCR1 includes CDR β1, CDR β2, and CDR β3. The amino acid sequence of CDR α3 of the T cell receptor LMP1-TCR1 is as shown in SEQ ID NO. 15, and the amino acid sequence of CDR β3 of the T cell receptor LMP1-TCR1 is as shown in SEQ ID NO. 11.

In some embodiments, in the TCR α-chain variable region of the T cell receptor LMP1-TCR1, the amino acid sequence of CDR α1 is as shown in SEQ ID NO. 13, or the amino acid sequence of CDR α2 is as shown in SEQ ID NO. 14.

In some embodiments, in the TCR β-chain variable region of the T cell receptor LMP1-TCR1, the amino acid sequence of CDR β1 is as shown in SEQ ID NO. 9, or the amino acid sequence of CDR β2 is as shown in SEQ ID NO. 10.

The present disclosure further provides a T cell receptor LMP1-TCR2 or a fragment thereof. The TCR α-chain variable region of the T cell receptor LMP1-TCR2 includes CDR α1, CDR α2, and CDR α3, and the TCR β-chain variable region of the T cell receptor LMP1-TCR2 includes CDR β1, CDR β2, and CDR β3. The amino acid sequence of CDR α3 of the T cell receptor LMP1-TCR2 is as shown in SEQ ID NO. 23, and the amino acid sequence of CDR β3 of the T cell receptor LMP1-TCR2 is as shown in SEQ ID NO. 19.

In some embodiments, in the TCR α-chain variable region of the T cell receptor LMP1-TCR2, the amino acid sequence of CDR α1 is as shown in SEQ ID NO. 21, or the amino acid sequence of CDR α2 is as shown in SEQ ID NO. 22.

In some embodiments, in the TCR β-chain variable region of the T cell receptor LMP1-TCR2, the amino acid sequence of CDR β1 is as shown in SEQ ID NO. 17, or the amino acid sequence of CDR β2 is as shown in SEQ ID NO. 18.

The present disclosure further provides a T cell receptor EBNA-3B-TCR or a fragment thereof. The TCR α-chain variable region of the T cell receptor EBNA-3B-TCR includes CDR α1, CDR α2, and CDR α3, and the TCR β-chain variable region of the T cell receptor EBNA-3B-TCR includes CDR β1, CDR β2, and CDR β3. The amino acid sequence of CDR α3 of the T cell receptor EBNA-3B-TCR is as shown in SEQ ID NO. 33, and the amino acid sequence of CDR β3 of the T cell receptor EBNA-3B-TCR is as shown in SEQ ID NO. 29.

In some embodiments, in the TCR α-chain variable region of the T cell receptor EBNA-3B-TCR, the amino acid sequence of CDR α1 is as shown in SEQ ID NO. 31, or the amino acid sequence of CDR α2 is as shown in SEQ ID NO. 32.

In some embodiments, in the TCR β-chain variable region of the T cell receptor EBNA-3B-TCR, the amino acid sequence of CDR β1 is as shown in SEQ ID NO. 27, or the amino acid sequence of CDR β2 is as shown in SEQ ID NO. 28.

The present disclosure further provides a T cell receptor LMP2-TCR or a fragment thereof. The TCR α-chain variable region of the T cell receptor LMP2-TCR includes CDR α1, CDR α2, and CDR α3, and the TCR β-chain variable region of the T cell receptor LMP2-TCR includes CDR β1, CDR β2, and CDR β3. The amino acid sequence of CDR α3 of the T cell receptor LMP2-TCR is as shown in SEQ ID NO. 42, and the amino acid sequence of CDR β3 of the T cell receptor LMP2-TCR is as shown in SEQ ID NO. 38.

In some embodiments, in the TCR α-chain variable region of the T cell receptor LMP2-TCR, the amino acid sequence of CDR α1 is as shown in SEQ ID NO. 40, or the amino acid sequence of CDR α2 is as shown in SEQ ID NO. 41.

In some embodiments, in the TCR β-chain variable region of the T cell receptor LMP2-TCR, the amino acid sequence of CDR β1 is as shown in SEQ ID NO. 36, or the amino acid sequence of CDR β2 is as shown in SEQ ID NO. 37.

In some embodiments, the HLA subtypes targeted by the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR include HLA-A*02:01 and HLA-A*11:01. More specifically, the HLA subtype targeted by the foregoing T cell receptor BMLF1-TCR, the foregoing T cell receptor LMP 1-TCR1, or the foregoing T cell receptor LMP1-TCR2 is HLA-A*02:01; or, the HLA subtype targeted by the foregoing T cell receptor EBNA-3B-TCR is HLA-A*11:01; or, the HLA subtype targeted by the foregoing T cell receptor LMP2-TCR is HLA-A*02:01.

A complementarity determining region (CDR) generally refers to a region of an antibody or receptor that is spatially capable of forming complementarity with an antigenic determinant. Variability within an antibody or receptor is typically not evenly distributed throughout the entire variable region. The α-chain variable region and the β-chain variable region of a monoclonal antibody or receptor each typically include three hypervariable regions (HVRs). These regions are generally capable of forming complementarity with antigenic determinants in spatial structure, and therefore the hypervariable regions are also referred to as complementarity determining regions (CDRs). Accordingly, the α-chain variable region typically includes three CDRs, namely CDR α1, CDR α2, and CDR α3, and the β-chain variable region typically also includes three CDRs, namely CDR β1, CDR β2, and CDR β3.

In some embodiments, the α-chain variable region and the β-chain variable region may further include framework regions. The framework regions may be located between the complementarity determining regions or at the termini of the complementarity determining regions. In certain embodiments of the present disclosure, the framework region sequence is obtained by substitution, deletion, or addition of one or more amino acids (specifically, 1-50, 1-30, 1-20, 1-10, 1-5, or 1-3 amino acids) based on the framework region sequence of a human monoclonal antibody variable region or a murine monoclonal antibody variable region. The framework region sequence may have at least 80%, 85%, 90%, 93%, 95%, 97%, or 99% sequence identity to a framework region sequence of a human monoclonal antibody variable region.

In some embodiments, the amino acid sequence of the TCR α-chain variable region of the T cell receptor BMLF1-TCR is as shown in SEQ ID NO. 45; and/or, the amino acid sequence of the TCR β-chain variable region of the T cell receptor BMLF 1-TCR is as shown in SEQ ID NO. 46.

In some embodiments, the amino acid sequence of the TCR α-chain variable region of the T cell receptor LMP1-TCR1 is as shown in SEQ ID NO. 47; and/or, the amino acid sequence of the TCR β-chain variable region of the T cell receptor LMP1-TCR1 is as shown in SEQ ID NO. 48.

In some embodiments, the amino acid sequence of the TCR α-chain variable region of the T cell receptor LMP1-TCR2 is as shown in SEQ ID NO. 49; and/or, the amino acid sequence of the TCR β-chain variable region of the T cell receptor LMP1-TCR2 is as shown in SEQ ID NO. 50.

In some embodiments, the amino acid sequence of the TCR α-chain variable region of the T cell receptor EBNA-3B-TCR is as shown in SEQ ID NO. 51; and/or, the amino acid sequence of the TCR β-chain variable region of the T cell receptor EBNA-3B-TCR is as shown in SEQ ID NO. 52.

In some embodiments, the amino acid sequence of the TCR α-chain variable region of the T cell receptor LMP2-TCR is as shown in SEQ ID NO. 53; and/or, the amino acid sequence of the TCR β-chain variable region of the T cell receptor LMP2-TCR is as shown in SEQ ID NO. 54.

In some embodiments, the TCR α-chain variable region may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 45, SEQ ID NO. 47, SEQ ID NO. 49, SEQ ID NO. 51, or SEQ ID NO. 53. The TCR β-chain variable region may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 46, SEQ ID NO. 48, SEQ ID NO. 50, SEQ ID NO. 52, or SEQ ID NO. 54.

In some embodiments, the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR further includes a TCR α-chain constant region and/or a TCR β-chain constant region.

In some embodiments, the amino acid sequence of the TCR α-chain constant region is as shown in SEQ ID NO. 55; and/or, the amino acid sequence of the TCR β-chain constant region is as shown in SEQ ID NO. 56.

In some embodiments, the TCR α-chain constant region may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 55; and the TCR β-chain constant region may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 56.

In some embodiments, the amino acid sequence of the TCR α-chain of the T cell receptor BMLF1-TCR is as shown in SEQ ID NO. 8, and the amino acid sequence of the TCR β-chain of the T cell receptor BMLF1-TCR is as shown in SEQ ID NO. 4.

In some embodiments, the amino acid sequence of the TCR α-chain of the T cell receptor LMP1-TCR1 is as shown in SEQ ID NO. 16, and the amino acid sequence of the TCR β-chain of the T cell receptor LMP1-TCR1 is as shown in SEQ ID NO. 12.

In some embodiments, the amino acid sequence of the TCR α-chain of the T cell receptor LMP1-TCR2 is as shown in SEQ ID NO. 24, and the amino acid sequence of the TCR β-chain of the T cell receptor LMP1-TCR2 is as shown in SEQ ID NO. 20.

In some embodiments, the amino acid sequence of the TCR α-chain of the T cell receptor EBNA-3B-TCR is as shown in SEQ ID NO. 34, and the amino acid sequence of the TCR β-chain of the T cell receptor EBNA-3B-TCR is as shown in SEQ ID NO. 30.

In some embodiments, the amino acid sequence of the TCR α-chain of the T cell receptor LMP2-TCR is as shown in SEQ ID NO. 43, and the amino acid sequence of the TCR β-chain of the T cell receptor LMP2-TCR is as shown in SEQ ID NO. 39.

In some embodiments, provided that the foregoing TCR α-chain and TCR β-chain variable regions include the aforementioned CDR sequences, the TCR α-chain may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 8, SEQ ID NO. 16, SEQ ID NO. 24, SEQ ID NO. 34, or SEQ ID NO. 43; and the TCR β-chain may alternatively be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as shown in SEQ ID NO. 4, SEQ ID NO. 12, SEQ ID NO. 20, SEQ ID NO. 30, or SEQ ID NO. 39.

In some embodiments, the TCR α-chain is a human TCR α-chain, a humanized TCR α-chain, a chimeric TCR α-chain, or a murine TCR α-chain; and the TCR β-chain is a human TCR β-chain, a humanized TCR β-chain, a chimeric TCR β-chain, or a murine TCR β-chain. As used herein, the term "chimeric TCR α-chain" or "chimeric TCR β-chain" means that the TCR α-chain or the TCR β-chain includes sequences derived from more than one species, for example, sequences derived from human and mouse.

In some embodiments, the T cell receptor BMLF1-TCR binds to a BMLF1 antigen; or the T cell receptor LMP1-TCR1 and the T cell receptor LMP1-TCR2 bind to an LMP1 antigen; or the T cell receptor EBNA-3B-TCR binds to an EBNA3 antigen; or the T cell receptor LMP2-TCR binds to an LMP2 antigen.

The present disclosure further provides an antigen-binding protein, wherein the antigen-binding protein includes the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR.

The present disclosure further provides an EBV antigen peptide, wherein the EBV antigen peptide includes an antigen peptide BMLF1, an antigen peptide LMP1, an antigen peptide EBNA3, or an antigen peptide LMP2.

In some embodiments, the amino acid sequence of the antigen peptide BMLF1 is as shown in SEQ ID NO. 25.

In some embodiments, the amino acid sequence of the antigen peptide LMP1 is as shown in SEQ ID NO. 26.

In some embodiments, the amino acid sequence of the antigen peptide EBNA3 is as shown in SEQ ID NO. 35.

In some embodiments, the amino acid sequence of the antigen peptide LMP2 is as shown in SEQ ID NO. 44.

In some embodiments, the foregoing antigen peptide BMLF1 is recognizable by the foregoing T cell receptor BMLF1-TCR; or the foregoing antigen peptide LMP1 is recognizable by the foregoing T cell receptor LMP1-TCR1 or the foregoing T cell receptor LMP1-TCR2; or the foregoing antigen peptide EBNA3 is recognizable by the foregoing T cell receptor EBNA-3B-TCR; or the foregoing antigen peptide LMP2 is recognizable by the foregoing T cell receptor LMP2-TCR.

The present disclosure further provides a method for preparing the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR, wherein the preparation method includes the following steps:
1) loading the foregoing antigen peptide BMLF1, the foregoing antigen peptide LMP1, the foregoing antigen peptide EBNA3, or the foregoing antigen peptide LMP2 onto *in vitro*-differentiated dendritic cells (DCs); and
2) co-culturing the dendritic cells obtained in step 1) with CD8+ T cells, thereby obtaining the T cell receptor BMLF1-TCR, the T cell receptor LMP1-TCR1, the T cell receptor LMP1-TCR2, the T cell receptor EBNA-3B-TCR, or the T cell receptor LMP2-TCR.

In some embodiments, the loading in step 1) includes mixing and incubating the antigen peptide BMLF1, the antigen peptide LMP1, the antigen peptide EBNA3, or the antigen peptide LMP2 with mature dendritic cells, followed by irradiation.

The present disclosure further provides a biological material, wherein the biological material includes a polynucleotide, a nucleic acid construct, a virus, or a cell.

In some embodiments, the polynucleotide encodes the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR; or the polynucleotide encodes the foregoing antigen peptide BMLF1, the foregoing antigen peptide LMP1, the foregoing antigen peptide EBNA3, or the foregoing antigen peptide LMP2.

In some embodiments, the nucleic acid construct includes the foregoing polynucleotide and a plasmid backbone.

In some embodiments, the plasmid backbone is a lentiviral, adenoviral, or adeno-associated viral plasmid backbone. More specifically, the plasmid backbone is one or more of pLKO.1-CMV-tGFP, pLKO.1-puro-CMV-tGFP, pLKO.1-CMV-Neo, pLKO.1-Neo, pLKO.1-Neo-CMV-tGFP, pLKO.1-puro-CMV-TagCFP, pLKO.1-puro-CMV-TagYFP, pLKO.1-puro-CMV-TagRFP, pLKO.1-puro-CMV-TagFP635, pLKO.1-puro-UbC-TurboGFP, pLKO.1-puro-UbC-TagFP635, pLKO-puro-IPTG-lxLacO, pLKO-puro-IPTG-3xLacO, pLP1, pLP2, pLP/VSV-G, pENTR/U6, pLenti6/BLOCK-iT-DEST, pcDNA1.2/V5-GW/lacZ, pLenti6.2/N-Lumio/V5-DEST, pGCSIL-GFP, and pHR-SFFV-Puro.

In some embodiments, the virus includes the foregoing polynucleotide.

In some embodiments, the virus is a lentivirus, an adenovirus, or an adeno-associated virus.

In some embodiments, the cell includes the foregoing T cell receptor BMLF1-TCR, the foregoing T cell receptor LMP1-TCR1, the foregoing T cell receptor LMP1-TCR2, the foregoing T cell receptor EBNA-3B-TCR, or the foregoing T cell receptor LMP2-TCR, the foregoing polynucleotide, the foregoing nucleic acid construct, or the foregoing virus.

In some embodiments, the cell is a cell in which the foregoing polynucleotide is genomically integrated.

In some embodiments, the cell is a T cell. Preferably, the T cell is a TCR-T cell.

In some embodiments, the T cell is further a helper T cell, a suppressor T cell, an effector T cell, a cytotoxic T cell, a delayed-type hypersensitivity T cell, a natural T cell, or a memory T cell. Herein, the helper T cell refers to a T cell having functions of assisting humoral immunity and cellular immunity; the suppressor T cell refers to a T cell having functions of suppressing cellular immunity and humoral immunity; the effector T cell refers to a T cell having the function of releasing lymphokines; the cytotoxic T cell refers to a T cell having the function of killing target cells; the delayed-type hypersensitivity T cell refers to a T cell participating in type IV hypersensitivity reactions and capable of acting on helper T cells and suppressor T cells to enhance immune effects; the natural T cell refers to an undifferentiated T cell; and the memory T cell refers to a T cell having the ability to remember specific antigenic stimulation.

In some embodiments, the T cell is a Jurkat T cell or a CD8+ T cell.

The present disclosure further provides a use of the foregoing T cell receptor BMLF1-TCR, the foregoing T cell receptor LMP1-TCR1, the foregoing T cell receptor LMP1-TCR2, the foregoing T cell receptor EBNA-3B-TCR, the foregoing T cell receptor LMP2-TCR, the foregoing polynucleotide, the foregoing nucleic acid construct, the foregoing virus, or the foregoing cell in the preparation of a tumor therapeutic product.

The present disclosure further provides a use of the foregoing antigen peptide BMLF1, the foregoing antigen peptide LMP1, the foregoing antigen peptide EBNA3, the foregoing antigen peptide LMP2, the foregoing polynucleotide, or the foregoing nucleic acid construct in the preparation of an anti-tumor peptide vaccine product.

The present disclosure further provides a use of the foregoing antigen peptide BMLF1, the foregoing antigen peptide LMP1, the foregoing antigen peptide EBNA3, the foregoing antigen peptide LMP2, the foregoing polynucleotide, or the foregoing nucleic acid construct in the preparation of a product for generating the T cell receptor *in vitro.*

In some embodiments, the tumor is one or more of nasopharyngeal carcinoma, adrenocortical carcinoma, bladder urothelial carcinoma, breast cancer, cervical squamous cell carcinoma, cervical adenocarcinoma, cholangiocarcinoma, colon adenocarcinoma, lymphoid tumors, esophageal cancer, glioblastoma multiforme, head and neck squamous cell carcinoma, chromophobe renal cell carcinoma, clear cell renal cell carcinoma, papillary renal cell carcinoma, acute myeloid leukemia, low-grade glioma, hepatocellular carcinoma, mesothelioma, ovarian cancer, pancreatic cancer, pheochromocytoma, paraganglioma, prostate cancer, rectal cancer, malignant sarcoma, melanoma, gastric cancer, testicular germ cell tumor, thyroid cancer, thymic carcinoma, endometrial cancer, chronic myeloid leukemia, lung cancer, anal cancer, and retinoblastoma.

In some embodiments, the lymphoid tumor includes natural killer T (NKT) cell lymphoma, Hodgkin lymphoma, or diffuse large B-cell lymphoma.

The present disclosure further provides a tumor therapeutic product, wherein the tumor therapeutic product includes the foregoing T cell receptor BMLF1-TCR, the foregoing T cell receptor LMP1-TCR1, the foregoing T cell receptor LMP1-TCR2, the foregoing T cell receptor EBNA-3B-TCR, the foregoing T cell receptor LMP2-TCR, the foregoing EBV antigen peptide, the foregoing polynucleotide, the foregoing nucleic acid construct, the foregoing virus, or the foregoing T cell, and a pharmaceutically acceptable auxiliary material.

In some embodiments, the auxiliary material includes various excipients and diluents that are not essential active ingredients and do not cause undue toxicity upon administration. The auxiliary material includes sterile water or normal saline, stabilizers, excipients, antioxidants (such as ascorbic acid), buffers (such as phosphates, citrates, or other organic acids), preservatives, surfactants (such as PEG or Tween), chelating agents (such as EDTA), or binders. The auxiliary material further includes other low-molecular-weight polypeptides, serum albumin, glycine, glutamine, asparagine, arginine, polysaccharides, monosaccharides, mannitol, or sorbitol. When used in aqueous solution for injection, the auxiliary material is normal saline, isotonic glucose solution, isotonic D-sorbitol solution, isotonic D-mannitol solution, or isotonic D-sugar alcohol solution. The aqueous solution for injection further includes a solubilizer. The solubilizer is an alcohol (such as ethanol), a polyol (such as propylene glycol or PEG), and/or a non-ionic surfactant (such as Tween 80 or HCO-50). In the tumor therapeutic product provided by the present disclosure, the foregoing TCR, the foregoing polynucleotide, the foregoing nucleic acid construct, or the foregoing T cell may be used as a single active ingredient, or may be combined with one or more additional active components useful for tumor treatment to form a combination formulation. The additional active component(s) are other various drugs used for tumor treatment. The content of the active component(s) in the pharmaceutical composition is a safe and effective amount, which can be adjusted by a person skilled in the art. For example, the administration dosage of the foregoing TCR, the foregoing polynucleotide, the foregoing nucleic acid construct, or the foregoing T cell, or of the active ingredient of the tumor therapeutic product, depends on factors such as the patient's body weight, the type of administration, and the condition and severity of the disease. For instance, the administration dosage of the active ingredient may be 1-1000 mg/kg/day, 1-3 mg/kg/day, 3-5 mg/kg/day, 5-10 mg/kg/day, 10-20 mg/kg/day, 20-30 mg/kg/day, 30-40 mg/kg/day, 40-60 mg/kg/day, 60-80 mg/kg/day, 80-100 mg/kg/day, 100-200 mg/kg/day, 200-500 mg/kg/day, or greater than 500 mg/kg/day.

The present disclosure further provides a method for treating a tumor, including administering the foregoing T cell receptor BMLF1-TCR, the foregoing T cell receptor LMP1-TCR1, the foregoing T cell receptor LMP1-TCR2, the foregoing T cell receptor EBNA-3B-TCR, the foregoing T cell receptor LMP2-TCR, the foregoing polynucleotide, the foregoing nucleic acid construct, the foregoing virus, or the foregoing T cell to a tumor patient.

In some embodiments, the administration dosage is 1-1000 mg/kg/day. Specifically, the administration dosage is 1-3 mg/kg/day, 3-5 mg/kg/day, 5-10 mg/kg/day, 10-20 mg/kg/day, 20-30 mg/kg/day, 30-40 mg/kg/day, 40-60 mg/kg/day, 60-80 mg/kg/day, 80-100 mg/kg/day, 100-200 mg/kg/day, 200-500 mg/kg/day, or 500-1000 mg/kg/day.

In some embodiments, the subject of the method is a mammal; preferably, the subject is a human.

In the present disclosure, a T cell receptor (TCR) is a molecule present on the surface of a T cell and is responsible for recognizing a peptide-major histocompatibility complex (peptide-MHC). In some embodiments, the TCR is a truncated TCR or a full-length TCR. In some embodiments, the TCR is a heterodimer composed of an α-chain and a β-chain. In some embodiments, the TCR may also be a single-chain TCR (scTCR).

In the present disclosure, the term "TCR α-chain constant region" or "TCR β-chain constant region" includes, in sequence, an extracellular constant region, a transmembrane region, and an intracellular constant region. The extracellular constant region may include the hinge region of the TCR α-chain or the TCR β-chain, which participates in the formation of a disulfide bond between the TCR α-chain and the TCR β-chain. The transmembrane region is also part of the constant region and functions to anchor the TCR α-chain and TCR β-chain to the cell membrane and to interact with CD3 subunits, thereby forming a TCR-CD3 complex. The intracellular constant region may function in conformational changes of the TCR-CD3 complex and signal transduction following TCR activation.

In the present disclosure, the term "variable region" or "variable domain" refers to a domain of an immunoglobulin superfamily binding protein (for example, a TCR α-chain or β-chain (or TCR γ-chain or δ-chain)) that participates in the binding of the immunoglobulin superfamily binding protein (for example, a TCR) to the antigen. The variable domains of the α-chain and β-chain of a native TCR (designated Vα and Vβ, respectively) typically have similar structures, and each variable domain contains four conserved framework regions (FRs) and three complementarity-determining regions (CDRs). The Vα domain is encoded by two independent DNA segments, namely a variable gene segment (V) and a joining gene segment (J), whereas the Vβ domain is encoded by three independent DNA segments, namely a variable gene segment (V), a diversity gene segment (D), and a joining gene segment (J). A single Vα or Vβ domain may be sufficient to confer antigen-binding specificity. In addition, for a TCR that binds a specific antigen, a Vα or Vβ domain may be isolated from the antigen-binding TCR and used to screen a library for a complementary Vα or Vβ domain, respectively.

In the present disclosure, the term "antigen" refers to a molecule that is present on the cell surface or intracellularly and presented by an MHC molecule or an MHC-like molecule, and that can be bound by an antibody or a T cell receptor (TCR). The antigen includes, but is not limited to, a polypeptide antigen (such as NYESO-1, AFP, and MART-1), a lipid antigen (such as β-GlcCer, eLPA, and LPE), or a polysaccharide antigen (such as CA199, CA72-4, and CA125). The antigen may be a tumor antigen, such as a tumor-associated antigen (TAA) or a tumor-specific antigen (TSA).

In the present disclosure, the term "isolated" refers to a material that has been separated from its natural environment or otherwise subjected to human manipulation, such as the α-chain, β-chain, T cell receptor, or nucleic acid described herein. An isolated material may be essentially or substantially free of components that normally accompany it in its natural state, or may be present in an artificial state together with components that normally accompany it in its natural state. An isolated material may be in a natural, chemically synthesized, or recombinant form. An isolated material may also, or alternatively, be in an enriched, partially purified, or purified form.

In the present disclosure, the term "polynucleotide," also referred to as "nucleotide" or "nucleic acid," refers to a nucleic acid chain composed of deoxyribonucleic acid (DNA), ribonucleic acid (RNA), modified nucleic acids or bases, and/or analogs thereof, or any substrate that can be incorporated into a chain by a DNA or RNA polymerase.

In the present disclosure, the term "nucleic acid construct," also referred to as a "vector," refers to a construct capable of delivering one or more genes or sequences of interest into a host cell and preferably expressing the genes or sequences in the host cell. Examples of vectors include, but are not limited to, viral vectors, plasmids, cosmids, or bacteriophage vectors.

In the present disclosure, the term "host cell" refers to a cell into which exogenous nucleic acid has been introduced, including progeny of such a cell.

In the present disclosure, for the purpose of determining the percentage identity between two amino acid sequences or two nucleic acid sequences, the sequences are aligned for optimal comparison purposes (for example, gaps may be introduced into one or both of the first and second amino acid sequences or nucleic acid sequences for optimal alignment, or non-homologous sequences may be disregarded for comparison purposes). In a preferred embodiment, for comparison purposes, the length of the sequence to be aligned is at least 30%, preferably at least 40%, more preferably at least 50%, 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at that position.

Mathematical algorithms may be used to perform sequence comparisons and to calculate the percentage identity between two sequences. In a preferred embodiment, the Needleman-Wunsch algorithm ((1970) J. Mol. Biol. 48:444-453), as implemented in the GAP program integrated in the GCG software package (available at http://www.gcg.com), is used to determine the percentage identity between two amino acid sequences, employing the Blosum 62 matrix or the PAM250 matrix with gap penalties of 16, 14, 12, 10, 8, 6, or 4 and gap extension penalties of 1, 2, 3, 4, 5, or 6. In another preferred embodiment, the GAP program of the GCG software package (available at http://www.gcg.com) is used to determine the percentage identity between two nucleic acid sequences, employing the NWSgapdna.CMP matrix with gap penalties of 40, 50, 60, 70, or 80 and gap extension penalties of 1, 2, 3, 4, 5, or 6. A particularly preferred set of parameters (and the parameter set to be used unless otherwise specified) employs the Blosum 62 scoring matrix with a gap penalty of 12, a gap extension penalty of 4, and a frameshift penalty of 5.

Alternatively, the percentage identity between two amino acid sequences or two nucleic acid sequences may be determined using the PAM120 weighted residue table with a gap extension penalty of 12 and a gap penalty of 4, utilizing the E. Myers and W. Miller algorithm ((1989) CABIOS 4:11-17), as incorporated into the ALIGN program (version 2.0).

The following specific examples are provided to illustrate embodiments of the present disclosure. Those skilled in the art can readily understand other advantages and effects of the present disclosure based on the disclosure herein. The present disclosure may also be implemented or applied through other different specific embodiments, and various modifications or alterations may be made to the details of this specification from different viewpoints and applications without departing from the spirit of the present disclosure.

Before describing the specific embodiments of the present disclosure in further detail, it should be understood that the scope of protection of the present disclosure is not limited to the specific embodiments described below. It should also be understood that the terminology used in the embodiments of the present disclosure is for the purpose of describing particular embodiments only and is not intended to limit the scope of the present disclosure. In the specification and claims of the present disclosure, unless otherwise expressly indicated, the singular forms "a," "an," and "the" include plural forms.

Where numerical ranges are provided in the embodiments, it should be understood that, unless otherwise stated, both endpoints of each numerical range and any numerical value between the two endpoints may be selected. Unless otherwise defined, all technical and scientific terms used herein have the same meanings as commonly understood by those skilled in the art. In addition to the specific methods, devices, and materials described in the embodiments, any methods, devices, or materials of the prior art that are similar or equivalent to those described herein may also be used to implement the present disclosure, based on the knowledge of those skilled in the art and the disclosure of the present disclosure.

The sequences of the present disclosure are as follows:
SEQ ID No. 1
   SNHLY
SEQ ID No. 2
   FYNNEI
SEQ ID No. 3
   CASSEGQVSPGELFF
SEQ ID No. 4 
MDTWLVCWAIFSLLKAGLTEPEVTQTPSHQVTQMGQEVILRCVPISNHLYF
YWYRQILGQKVEFLVSFYNNEISEKSEIFDDQFSVERPDGSNFTLKIRSTKLEDSAM
YFCASSEGQVSPGELFFGEGSRLTVLEDLRNVTPPKVSLFEPSKAEIANKQKATLVC
LARGFFPDHVELSWWVNGKEVHSGVCTDPQAYKESNYSYCLSSRLRVSATFWHN
PRNHFRCQVQFHGLSEEDKWPEGSPKPVTQNISAEAWGRADCGITSASYQQGVLS
ATILYEILLGKATLYAVLVSTLVVMAMVKRKNS
SEQ ID No. 5
   NSASQS
SEQ ID No. 6
   VYSSG
SEQ ID No. 7
   CVVNGMDSSYKLIF
SEQ ID No. 8 
MISLRVLLVILWLQLSWVWSQRKEVEQDPGPFNVPEGATVAFNCTYSNSA
SQSFFWYRQDCRKEPKLLMSVYSSGNEDGRFTAQLNRASQYISLLIRDSKLSDSAT
YLCVVNGMDSSYKLIFGSGTRLLVRPDIQNPEPAVYQLKDPRSQDSTLCLFTDFDS
QINVPKTMESGTFITDKCVLDMKAMDSKSNGAIAWSNQTSFTCQDIFKETNATYP
SSDVPCDATLTEKSFETDMNLNFQNLSVMGLRILLLKVAGFNLLMTLRLWSS
SEQ ID No. 9
   MGHRA
SEQ ID No. 10
   YSYEKL
SEQ ID No. 11
   CASSQEGTGELFF
SEQ ID No. 12 
MGCRLLCCAVLCLLGAVPIDTEVTQTPKHLVMGMTNKKSLKCEQHMGHR
AMYWYKQKAKKPPELMFVYSYEKLSINESVPSRFSPECPNSSLLNLHLHALQPED
SALYLCASSQEGTGELFFGEGSRLTVLEDLRNVTPPKVSLFEPSKAEIANKQKATLV
CLARGFFPDHVELSWWVNGKEVHSGVCTDPQAYKESNYSYCLSSRLRVSATFWH
NPRNHFRCQVQFHGLSEEDKWPEGSPKPVTQNISAEAWGRADCGITSASYQQGVL
SATILYEILLGKATLYAVLVSTLVVMAMVKRKNS
SEQ ID No. 13
   YGGTVN
SEQ ID No. 14
   YFSGDPLV
SEQ ID No. 15
   CALPRWDGGYNKLIF
SEQ ID No. 16 
MLLLLIPVLGMIFALRDARAQSVSQHNHHVILSEAASLELGCNYSYGGTVN
LFWYVQYPGQHLQLLLKYFSGDPLVKGIKGFEAEFIKSKFSFNLRKPSVQWSDTA
EYFCALPRWDGGYNKLIFGAGTRLAVHPYYIQNPEPAVYQLKDPRSQDSTLCLFT
DFDSQINVPKTMESGTFITDKCVLDMKAMDSKSNGAIAWSNQTSFTCQDIFKETN
ATYPSSDVPCDATLTEKSFETDMNLNFQNLSVMGLRILLLKVAGFNLLMTLRLWS
S
SEQ ID No. 17
   DFQATT
SEQ ID No. 18
   SNEGSKA
SEQ ID No. 19
   CSASGAQPDDPQHF
SEQ ID No. 20 
MLLLLLLLGPGSGLGAVVSQHPSWVICKSGTSVKIECRSLDFQATTMFWY
RQFPKQSLMLMATSNEGSKATYEQGVEKDKFLINHASLTLSTLTVTSAHPEDSSFY
ICSASGAQPDDPQHFGDGTRLSILEDLRNVTPPKVSLFEPSKAEIANKQKATLVCL
ARGFFPDHVELSWWVNGKEVHSGVCTDPQAYKESNYSYCLSSRLRVSATFWHNP
 
RNHFRCQVQFHGLSEEDKWPEGSPKPVTQNISAEAWGRADCGITSASYQQGVLSA
TILYEILLGKATLYAVLVSTLVVMAMVKRKNS
SEQ ID No. 21
   TSDPSYG
SEQ ID No. 22
   QGSYDQQN
SEQ ID No. 23
   CAMREV ALNAGGTSYGKL TF
SEQ ID No. 24 
MSLSSLLKVVTASLWLGPGIAQKITQTQPGMFVQEKEAVTLDCTYDTSDPS
YGLFWYKQPSSGEMIFLIYQGSYDQQNATEGRYSLNFQKARKSANLVISASQLGD
SAMYFCAMREVALNAGGTSYGKLTFGQGTILTVHPNIQNPEPAVYQLKDPRSQDS
TLCLFTDFDSQINVPKTMESGTFITDKCVLDMKAMDSKSNGAIAWSNQTSFTCQDI
FKETNATYPSSDVPCDATLTEKSFETDMNLNFQNLSVMGLRILLLKVAGFNLLMT
LRLWSS
SEQ ID No. 25
   GLCTLVAML
SEQ ID No. 26
   YLLEMLWRL
SEQ ID No. 27
   SGHTA
SEQ ID No. 28
   FQGNSA
SEQ ID No. 29
   CASSLNGGHYEQYF
SEQ ID No. 30 
MGTRLLFWVAFCLLGADHTGAGVSQSPSNKVTEKGKDVELRCDPISGHTA
LYWYRQSLGQGLEFLIYFQGNSAPDKSGLPSDRFSAERTGGSVSTLTIQRTQQEDS
AVYLCASSLNGGHYEQYFGPGTRLTVTEDLRNVTPPKVSLFEPSKAEIANKQKAT
LVCLARGFFPDHVELSWWVNGKEVHSGVCTDPQAYKESNYSYCLSSRLRVSATF
WHNPRNHFRCQVQFHGLSEEDKWPEGSPKPVTQNISAEAWGRADCGITSASYQQ
GVLSATILYEILLGKATLYAVLVSTLVVMAMVKRKNS
SEQ ID No. 31
   SIFNT
SEQ ID No. 32
   LYKAGEL
SEQ ID No. 33
   CAGHRTDKLIF
SEQ ID No. 34 
MLLEHLLIILWMQLTWVSGQQLNQSPQSMFIQEGEDVSMNCTSSSIFNTWL
WYKQDPGEGPVLLIALYKAGELTSNGRLTAQFGITRKDSFLNISASIPSDVGIYFCA
GHRTDKLIFGTGTRLQVFPNIQNPEPAVYQLKDPRSQDSTLCLFTDFDSQINVPKT
MESGTFITDKCVLDMKAMDSKSNGAIAWSNQTSFTCQDIFKETNATYPSSDVPCD
 ATLTEKSFETDMNLNFQNLSVMGLRILLLKVAGFNLLMTLRLWSS
SEQ ID No. 35
   IVTDFSVIK
SEQ ID No. 36
   MNHEY
SEQ ID No. 37
   SVGAGI
SEQ ID No. 38
   CASSTQGGGDGYTF
SEQ ID No. 39 
MSIGLLCCAALSLLWAGPVNAGVTQTPKFQVLKTGQSMTLQCAQDMNHE
YMSWYRQDPGMGLRLIHYSVGAGITDQGEVPNGYNVSRSTTEDFPLRLLSAAPSQ
TSVYFCASSTQGGGDGYTFGSGTRLTVVEDLRNVTPPKVSLFEPSKAEIANKQKA
TLVCLARGFFPDHVELSWWVNGKEVHSGVCTDPQAYKESNYSYCLSSRLRVSAT
FWHNPRNHFRCQVQFHGLSEEDKWPEGSPKPVTQNISAEAWGRADCGITSASYQ
QGVLSATILYEILLGKATLYAVLVSTLVVMAMVKRKNS
SEQ ID No. 40
   TSINN
SEQ ID No. 41
   IRSNERE
SEQ ID No. 42
   CATEGDGGYSTLTF
SEQ ID No. 43 
METLLGVSLVILWLQLARVNSQQGEEDPQALSIQEGENATMNCSYKTSIN
NLQWYRQNSGRGLVHLILIRSNEREKHSGRLRVTLDTSKKSSSLLITASRAADTAS
YFCATEGDGGYSTLTFGKGTMLLVSPDIQNPEPAVYQLKDPRSQDSTLCLFTDFDS
QINVPKTMESGTFITDKCVLDMKAMDSKSNGAIAWSNQTSFTCQDIFKETNATYP
SSDVPCDATLTEKSFETDMNLNFQNLSVMGLRILLLKVAGFNLLMTLRLWSS
SEQ ID No. 44
   FLYALALLL
SEQ ID No. 45 
MISLRVLLVILWLQLSWVWSQRKEVEQDPGPFNVPEGATVAFNCTYSNSA
SQSFFWYRQDCRKEPKLLMSVYSSGNEDGRFTAQLNRASQYISLLIRDSKLSDSAT
YLCVVNGMDSSYKLIFGSGTRLLVRPD
SEQ ID No. 46 
MDTWLVCWAIFSLLKAGLTEPEVTQTPSHQVTQMGQEVILRCVPISNHLYF
YWYRQILGQKVEFLVSFYNNEISEKSEIFDDQFSVERPDGSNFTLKIRSTKLEDSAM
YFCASSEGQVSPGELFFGEGSRLTVL
SEQ ID No. 47 
MLLLLIPVLGMIFALRDARAQSVSQHNHHVILSEAASLELGCNYSYGGTVN
LFWYVQYPGQHLQLLLKYFSGDPLVKGIKGFEAEFIKSKFSFNLRKPSVQWSDTA
EYFCALPRWDGGYNKLIFGAGTRLAVHPYY
SEQ ID No. 48 
MGCRLLCCAVLCLLGAVPIDTEVTQTPKHLVMGMTNKKSLKCEQHMGHR
AMYWYKQKAKKPPELMFVYSYEKLSINESVPSRFSPECPNSSLLNLHLHALQPED
SALYLCASSQEGTGELFFGEGSRLTVL
SEQ ID No. 49 
MSLSSLLKVVTASLWLGPGIAQKITQTQPGMFVQEKEAVTLDCTYDTSDPS
YGLFWYKQPSSGEMIFLIYQGSYDQQNATEGRYSLNFQKARKSANLVISASQLGD
SAMYFCAMREVALNAGGTSYGKLTFGQGTILTVHPN
SEQ ID No. 50 
MLLLLLLLGPGSGLGAVVSQHPSWVICKSGTSVKIECRSLDFQATTMFWY
RQFPKQSLMLMATSNEGSKATYEQGVEKDKFLINHASLTLSTLTVTSAHPEDSSFY
ICSASGAQPDDPQHFGDGTRLSIL
SEQ ID No. 51 
MLLEHLLIILWMQLTWVSGQQLNQSPQSMFIQEGEDVSMNCTSSSIFNTWL
WYKQDPGEGPVLLIALYKAGELTSNGRLTAQFGITRKDSFLNISASIPSDVGIYFCA
GHRTDKLIFGTGTRLQVFPN
SEQ ID No. 52 
MGTRLLFWVAFCLLGADHTGAGVSQSPSNKVTEKGKDVELRCDPISGHTA
LYWYRQSLGQGLEFLIYFQGNSAPDKSGLPSDRFSAERTGGSVSTLTIQRTQQEDS
AVYLCASSLNGGHYEQYFGPGTRLTVT
SEQ ID No. 53 
METLLGVSLVILWLQLARVNSQQGEEDPQALSIQEGENATMNCSYKTSIN
NLQWYRQNSGRGLVHLILIRSNEREKHSGRLRVTLDTSKKSSSLLITASRAADTAS
YFCATEGDGGYSTLTFGKGTMLLVSP
SEQ ID No. 54 
MSIGLLCCAALSLLWAGPVNAGVTQTPKFQVLKTGQSMTLQCAQDMNHE
YMSWYRQDPGMGLRLIHYSVGAGITDQGEVPNGYNVSRSTTEDFPLRLLSAAPSQ
TSVYFCASSTQGGGDGYTFGSGTRLTVV
SEQ ID No. 55 
IQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMNLN
FQNLSVMGLRILLLKVAGFNLLMTLRLWSS
SEQ ID No. 56 
EDLRNVTPPKVSLFEPSKAEIANKQKATLVCLARGFFPDHVELSWWVNGK
EVHSGVCTDPQAYKESNYSYCLSSRLRVSATFWHNPRNHFRCQVQFHGLSEEDK
WPEGSPKPVTQNISAEAWGRADCGITSASYQQGVLSATILYEILLGKATLYAVLVS
TLVVMAMVKRKNS
SEQ ID No. 57
   RAKRGSGATNFSLLKQAGDVEENPGP
SEQ ID No. 58 
ATGGATACATGGCTCGTATGCTGGGCAATTTTTTCTCTCCTGAAAGCCG
GGCTGACTGAGCCTGAGGTTACACAGACACCCAGCCACCAAGTAACTCAGAT
GGGACAAGAGGTCATCCTTCGGTGCGTGCCCATTTCAAACCACCTGTACTTCT
ACTGGTACCGCCAGATTCTGGGTCAGAAAGTGGAGTTTCTGGTGAGCTTCTAT
AACAATGAGATCTCCGAGAAATCAGAGATTTTTGACGACCAGTTCTCTGTGGA
ACGGCCGGATGGGAGCAATTTCACTCTGAAGATTAGGAGCACTAAGCTGGAA
GATTCCGCCATGTATTTCTGCGCTTCAAGTGAGGGTCAGGTCAGTCCAGGAGA
GCTGTTTTTTGGCGAGGGTAGTAGATTGACCGTCCTGGAGGATCTGAGAAATG
 
TGACTCCACCCAAGGTCTCCTTGTTTGAGCCATCAAAAGCAGAGATTGCAAAC
AAACAAAAGGCTACCCTCGTGTGCTTGGCCAGGGGCTTCTTCCCTGACCACGT
GGAGCTGAGCTGGTGGGTGAATGGCAAGGAGGTCCACAGTGGGGTCTGCACG
GACCCTCAGGCCTACAAGGAGAGCAATTATAGCTACTGCCTGAGCAGCCGCCT
GAGGGTCTCTGCTACCTTCTGGCACAATCCTCGCAACCACTTCCGCTGCCAAG
TGCAGTTCCATGGGCTTTCAGAGGAGGACAAGTGGCCAGAGGGCTCACCCAA
ACCTGTCACACAGAACATCAGTGCAGAGGCCTGGGGCCGAGCAGACTGTGGG
ATTACCTCAGCATCCTATCAACAAGGGGTCTTGTCTGCCACCATCCTCTATGAG
ATCCTGCTAGGGAAAGCCACCCTGTATGCTGTGCTTGTCAGTACACTGGTGGT
GATGGCTATGGTCAAAAGAAAGAATTCAAGAGCCAAAAGAGGTTCTGGCGCC
ACCAACTTCAGCCTGCTGAAGCAGGCCGGCGACGTGGAGGAGAACCCCGGCC
CCATGATAAGCCTCAGAGTGTTGTTGGTAATCTTGTGGCTGCAGCTTTCCTGGG
TTTGGTCTCAGCGGAAAGAGGTTGAGCAGGACCCAGGACCTTTTAATGTCCCA
GAGGGTGCAACAGTTGCTTTCAACTGCACCTACTCCAACAGTGCATCCCAGTC
CTTCTTTTGGTATCGCCAGGATTGCAGAAAGGAACCAAAACTGCTCATGAGCG
TTTACTCAAGTGGAAACGAGGACGGTCGATTCACAGCACAGCTGAATCGCGC
ATCACAGTACATAAGTCTTCTCATCAGGGACAGTAAGTTGTCTGATTCTGCCA
CATACCTGTGTGTGGTAAACGGAATGGATAGCAGTTACAAACTGATCTTCGGC
AGTGGAACAAGACTGCTGGTGAGGCCTGACATCCAGAACCCAGAACCTGCTG
TGTACCAGTTAAAAGATCCTCGGTCTCAGGACAGCACCCTCTGCCTGTTCACC
GACTTTGACTCCCAAATCAATGTGCCGAAAACCATGGAATCTGGAACGTTCAT
CACTGACAAATGCGTGCTGGACATGAAAGCTATGGATTCCAAGAGCAATGGG
GCCATTGCCTGGAGCAACCAGACAAGCTTCACCTGCCAAGATATCTTCAAAGA
GACCAACGCCACCTACCCCAGTTCAGACGTTCCCTGTGATGCCACGTTGACCG
AGAAAAGCTTTGAAACAGATATGAACCTAAACTTTCAAAACCTGTCAGTTATG
GGACTCCGAATCCTCCTGCTGAAAGTAGCGGGATTTAACCTGCTCATGACGCT
GAGGCTGTGGTCCAGTTGA
SEQ ID No. 59 
ATGGGATGTAGACTGCTGTGCTGCGCTGTCCTTTGCCTGCTCGGAGCAG
TACCAATCGACACCGAAGTGACCCAAACACCTAAACACCTCGTGATGGGCAT
GACAAATAAGAAAAGCCTCAAGTGCGAACAACACATGGGCCACAGAGCAATG
TACTGGTATAAGCAGAAAGCTAAGAAGCCTCCAGAGCTGATGTTTGTATACTC
ATACGAGAAGCTGAGCATTAACGAGAGCGTGCCTAGTAGGTTTTCACCAGAGT
GCCCTAATTCTTCCCTTTTGAACCTGCACCTGCATGCGTTGCAACCTGAAGACT
CCGCCTTGTACCTGTGTGCATCTTCCCAGGAGGGGACTGGAGAGTTGTTTTTCG
GAGAAGGAAGTAGACTGACCGTTCTGGAGGATCTGAGAAATGTGACTCCACC
CAAGGTCTCCTTGTTTGAGCCATCAAAAGCAGAGATTGCAAACAAACAAAAG
GCTACCCTCGTGTGCTTGGCCAGGGGCTTCTTCCCTGACCACGTGGAGCTGAG
CTGGTGGGTGAATGGCAAGGAGGTCCACAGTGGGGTCTGCACGGACCCTCAG
GCCTACAAGGAGAGCAATTATAGCTACTGCCTGAGCAGCCGCCTGAGGGTCTC
TGCTACCTTCTGGCACAATCCTCGCAACCACTTCCGCTGCCAAGTGCAGTTCCA
TGGGCTTTCAGAGGAGGACAAGTGGCCAGAGGGCTCACCCAAACCTGTCACA
CAGAACATCAGTGCAGAGGCCTGGGGCCGAGCAGACTGTGGGATTACCTCAG
CATCCTATCAACAAGGGGTCTTGTCTGCCACCATCCTCTATGAGATCCTGCTAG
GGAAAGCCACCCTGTATGCTGTGCTTGTCAGTACACTGGTGGTGATGGCTATG
GTCAAAAGAAAGAATTCAAGAGCCAAAAGAGGTTCTGGCGCCACCAACTTCA
GCCTGCTGAAGCAGGCCGGCGACGTGGAGGAGAACCCCGGCCCCATGTTGCT
CCTCCTTATCCCAGTGCTCGGAATGATCTTCGCGCTCCGCGACGCCCGAGCCC
AGTCTGTGTCTCAACATAATCATCATGTGATCTTGTCTGAGGCTGCTAGCTTGG
AGCTGGGATGTAACTACTCTTATGGTGGCACCGTGAATCTCTTTTGGTATGTGC
AGTACCCTGGCCAACATCTCCAACTCTTGCTTAAATACTTTAGCGGTGATCCAC
TGGTAAAAGGGATTAAGGGTTTCGAGGCCGAGTTCATCAAAAGTAAGTTCAG
CTTCAATCTGCGGAAGCCTAGCGTTCAGTGGAGTGATACTGCCGAATATTTCT
GTGCATTGCCACGCTGGGATGGAGGATATAATAAGCTGATTTTCGGAGCCGGT
ACACGCCTGGCCGTTCATCCATACTACATCCAGAACCCAGAACCTGCTGTGTA
CCAGTTAAAAGATCCTCGGTCTCAGGACAGCACCCTCTGCCTGTTCACCGACT
TTGACTCCCAAATCAATGTGCCGAAAACCATGGAATCTGGAACGTTCATCACT
 
GACAAATGCGTGCTGGACATGAAAGCTATGGATTCCAAGAGCAATGGGGCCA
TTGCCTGGAGCAACCAGACAAGCTTCACCTGCCAAGATATCTTCAAAGAGACC
AACGCCACCTACCCCAGTTCAGACGTTCCCTGTGATGCCACGTTGACCGAGAA
AAGCTTTGAAACAGATATGAACCTAAACTTTCAAAACCTGTCAGTTATGGGAC
TCCGAATCCTCCTGCTGAAAGTAGCGGGATTTAACCTGCTCATGACGCTGAGG
CTGTGGTCCAGTTGA
SEQ ID No. 60 
ATGCTGTTGCTGCTCCTGCTGCTCGGACCAGGAAGTGGATTGGGAGCCG
TGGTCAGTCAGCATCCTAGCTGGGTAATATGCAAGTCAGGCACCTCCGTGAAGA
TCGAGTGCAGGAGTCTTGATTTCCAGGCGACGACCATGTTTTGGTACCGCCAGT
TCCCGAAACAGAGCCTCATGCTGATGGCAACGTCAAACGAGGGTTCAAAGGCA
ACCTATGAACAGGGCGTGGAGAAGGACAAGTTCCTGATAAACCATGCATCCCT
GACCCTCAGCACTTTGACAGTTACTAGCGCTCACCCAGAGGATTCTAGTTTTTA
CATTTGCTCCGCTAGTGGAGCACAGCCCGATGATCCACAGCACTTTGGAGATGG
AACCAGACTCAGCATCCTCgaggatctgagaaatgtgactccacccaaggtctccttgtttgagccatcaaaagc
agagattgcaaacaaacaaaaggctaccctcgtgtgcttggccaggggcttcttccctgaccacgtggagctgagctggtgggtg
aatggcaaggaggtccacagtggggtcTgcacggaccctcaggcctacaaggagagcaattatagctactgcctgagcagcc
gcctgagggtctctgctaccttctggcacaatcctcgcaaccacttccgctgccaagtgcagttccatgggctttcagaggaggac
aagtggccagagggctcacccaaacctgtcacacagaacatcagtgcagaggcctggggccgagcagactgtgggattacctc
agcatcctatcaacaaggggtcttgtctgccaccatcctctatgagatcctgctagggaaagccaccctgtatgctgtgcttgtcagt
acactggtggtgatggctatggtcaaaagaaagaattcaAGAGCCAAAAGAGGTTCTGGCGCCACCA
ACTTCAGCCTGCTGAAGCAGGCCGGCGACGTGGAGGAGAACCCCGGCCCCAT
GTCACTTAGCTCTCTTCTTAAAGTAGTAACTGCTTCACTTTGGCTTGGCCCCGGT
ATTGCACAGAAGATCACCCAGACACAACCCGGCATGTTCGTGCAGGAAAAGGA
GGCTGTGACGCTCGACTGCACCTATGATACCTCCGACCCAAGTTACGGCCTGTT
CTGGTACAAGCAGCCTAGCTCAGGAGAGATGATTTTCCTGATCTACCAGGGGTC
ATATGACCAACAGAACGCAACAGAAGGGAGGTATAGCCTGAATTTCCAGAAGG
CCCGAAAGAGTGCCAACCTGGTGATCTCCGCTAGTCAGCTTGGAGACAGCGCA
 
ATGTACTTTTGCGCAATGCGCGAAGTGGCACTCAATGCCGGGGGAACTTCTTAT
GGCAAGCTGACTTTCGGACAGGGTACGATACTCACTGTCCATCCAAacatccagaacc
cagaacctgctgtgtaccagttaaaagatcctcggtctcaggacagcaccctctgcctgttcaccgactttgactcccaaatcaatgt
gccgaaaaccatggaatctggaacgttcatcactgacaaaTGCgtgctggacatgaaagctatggattccaagagcaatgggg
ccattgcctggagcaaccagacaagcttcacctgccaagatatcttcaaagagaccaacgccacctaccccagttcagacgttcc
ctgtgatgccacgttgaccgagaaaagctttgaaacagatatgaacctaaactttcaaaacctgtcagttatgggactccgaatcctc
ctgctgaaagtagcgggatttaacctgctcatgacgctgaggctgtggtccagtTGA
SEQ ID No. 61 
ATGGGAACACGCCTTCTGTTTTGGGTTGCCTTCTGCCTGTTGGGCGCTG
ACCACACCGGTGCAGGCGTCTCTCAGTCACCATCAAACAAGGTGACTGAGAAA
GGGAAAGATGTGGAGCTGAGATGTGACCCTATCTCTGGACACACCGCCCTGTA
CTGGTACAGACAGTCTCTTGGTCAGGGGCTGGAGTTCCTGATCTACTTTCAGGG
AAACAGCGCCCCAGACAAGTCTGGTCTCCCCTCTGACAGATTTTCCGCAGAAA
GGACCGGCGGTAGCGTGTCAACTCTGACCATACAGCGCACCCAGCAAGAAGAT
TCCGCCGTGTACCTGTGCGCCTCTTCACTCAACGGCGGCCATTATGAGCAGTAC
TTCGGACCTGGCACCCGCCTCACTGTGACCgaggatctgagaaatgtgactccacccaaggtctcctt
gtttgagccatcaaaagcagagattgcaaacaaacaaaaggctaccctcgtgtgcttggccaggggcttcttccctgaccacgtgg
agctgagctggtgggtgaatggcaaggaggtccacagtggggtcTgcacggaccctcaggcctacaaggagagcaattatag
ctactgcctgagcagccgcctgagggtctctgctaccttctggcacaatcctcgcaaccacttccgctgccaagtgcagttccatgg
gctttcagaggaggacaagtggccagagggctcacccaaacctgtcacacagaacatcagtgcagaggcctggggccgagca
gactgtgggattacctcagcatcctatcaacaaggggtcttgtctgccaccatcctctatgagatcctgctagggaaagccaccctg
tatgctgtgcttgtcagtacactggtggtgatggctatggtcaaaagaaagaattcaAGAGCCAAAAGAGGTTCT
GGCGCCACCAACTTCAGCCTGCTGAAGCAGGCCGGCGACGTGGAGGAGAACC
CCGGCCCCATGCTGCTTGAACACCTCCTGATTATTCTTTGGATGCAGCTGACTTG
GGTCAGTGGGCAGCAGCTGAATCAGAGCCCCCAGAGCATGTTCATTCAAGAAG
GGGAAGATGTGTCCATGAATTGCACCTCCTCCAGCATCTTCAACACCTGGCTTT
GGTACAAGCAAGACCCCGGAGAAGGACCAGTTCTCCTTATAGCCCTGTACAAG
GCCGGAGAGCTGACCAGTAATGGTCGACTGACCGCGCAATTCGGAATAACGCG
 
GAAGGACAGCTTCCTGAATATCAGCGCAAGCATTCCAAGTGATGTCGGAATCTA
TTTTTGCGCTGGACATCGAACCGATAAACTGATTTTCGGAACAGGGACCAGACT
GCAAGTGTTTCCCAacatccagaacccagaacctgctgtgtaccagttaaaagatcctcggtctcaggacagcaccc
tctgcctgttcaccgactttgactcccaaatcaatgtgccgaaaaccatggaatctggaacgttcatcactgacaaaTGCgtgctg
gacatgaaagctatggattccaagagcaatggggccattgcctggagcaaccagacaagcttcacctgccaagatatcttcaaag
agaccaacgccacctaccccagttcagacgttccctgtgatgccacgttgaccgagaaaagctttgaaacagatatgaacctaaac
tttcaaaacctgtcagttatgggactccgaatcctcctgctgaaagtagcgggatttaacctgctcatgacgctgaggctgtggtcca
gtTGA
SEQ ID No. 62

ATGAGCATAGGTCTCCTTTGTTGTGCCGCACTTAGCCTGCTTTGGGCCGG
CCCCGTTAACGCTGGTGTTACTCAAACGCCGAAGTTCCAGGTGCTCAAAACTG
GCCAGTCAATGACACTGCAGTGCGCTCAGGACATGAACCACGAATATATGAGTT
GGTACAGACAGGACCCTGGGATGGGATTGAGGCTGATTCACTACAGTGTCGGA
GCTGGGATCACAGACCAGGGCGAAGTCCCCAACGGCTACAACGTGTCAAGGTC
TACCACCGAGGACTTTCCCCTCAGACTTCTCTCTGCTGCACCATCTCAGACGAG
TGTGTACTTCTGTGCTAGCAGCACTCAAGGGGGCGGTGATGGCTACACGTTTGG
AAGTGGCACCCGGCTCACAGTGGTTgaggatctgagaaatgtgactccacccaaggtctccttgtttgagc
catcaaaagcagagattgcaaacaaacaaaaggctaccctcgtgtgcttggccaggggcttcttccctgaccacgtggagctgag
ctggtgggtgaatggcaaggaggtccacagtggggtcTgcacggaccctcaggcctacaaggagagcaattatagctactgcc
tgagcagccgcctgagggtctctgctaccttctggcacaatcctcgcaaccacttccgctgccaagtgcagttccatgggctttcag
aggaggacaagtggccagagggctcacccaaacctgtcacacagaacatcagtgcagaggcctggggccgagcagactgtg
ggattacctcagcatcctatcaacaaggggtcttgtctgccaccatcctctatgagatcctgctagggaaagccaccctgtatgctgt
gcttgtcagtacactggtggtgatggctatggtcaaaagaaagaattcaAGAGCCAAAAGAGGTTCTGGCGC
CACCAACTTCAGCCTGCTGAAGCAGGCCGGCGACGTGGAGGAGAACCCCGGC
CCCATGGAAACACTGCTCGGCGTGTCACTCGTTATTCTTTGGCTCCAGCTGGCC
CGGGTTAATTCACAACAGGGCGAGGAAGATCCTCAAGCGTTGTCTATTCAGGA
GGGAGAGAACGCAACCATGAACTGCAGCTATAAAACCTCTATCAACAACCTCC
AGTGGTATCGGCAGAACTCAGGCCGAGGTCTGGTCCATCTGATACTGATTCGCA

GCAATGAGCGCGAGAAGCATTCTGGGAGACTGCGCGTCACACTCGACACATCA
AAGAAAAGCTCCTCCCTGCTGATCACGGCTTCAAGAGCCGCAGACACTGCGAG
CTACTTCTGTGCAACTGAGGGCGATGGTGGGTATTCCACTCTCACATTTGGTAA
AGGGACCATGCTGCTGGTGTCCCCTGacatccagaacccagaacctgctgtgtaccagttaaaagatcctc
ggtctcaggacagcaccctctgcctgttcaccgactttgactcccaaatcaatgtgccgaaaaccatggaatctggaacgttcatca
ctgacaaaTGCgtgctggacatgaaagctatggattccaagagcaatggggccattgcctggagcaaccagacaagcttcacc
tgccaagatatcttcaaagagaccaacgccacctaccccagttcagacgttccctgtgatgccacgttgaccgagaaaagctttga
aacagatatgaacctaaactttcaaaacctgtcagttatgggactccgaatcctcctgctgaaagtagcgggatttaacctgctcatg
acgctgaggctgtggtccagtTGA

### Example 1 Sorting of TCR-T Cells and TCR Sequencing

### 1. In vitro differentiation of human peripheral blood mononuclear cells into dendritic cells (DCs)

(1) CD14+ monocytes were isolated from peripheral blood of healthy donors using magnetic beads (Miltenyi Biotec, Cat. No. 130-050-201) and cultured in AIM-V medium (Thermo) containing 1% human serum, human GM-CSF (Peprotech, 100 ng/mL), and IL-4 (50 ng/mL) for 3 days. The medium was then replaced, and the cells were further cultured for 2 days to induce differentiation of monocytes into monocyte-derived dendritic cells (MoDCs).
(2) After 5 days of differentiation, cytokines including TNF-α (10 ng/mL), IL-1β (10 ng/mL), IL-6 (10 ng/mL), and Prostaglandin E2 (Peprotech, 1 µg/mL) were added to induce maturation of DCs.
(3) After 24 h, mature DCs were harvested and analyzed by flow cytometry to detect the expression of mature DCs markers. The phenotype of mature DCs was as follows: CD14-, CD11c+, CD40+, CD80+, CD83+, CD86+, HLA-ABC+, and HLA-DR+. The obtained mature DCs were collected and used for subsequent loading of EBV antigen peptides.

### 2. Co-stimulatory culture of antigen peptide-loaded DCs with autologous CD8+ T cells

(1) 5 µg/mL of EBV antigen peptides (namely antigen peptide BMLF1, antigen peptide LMP1, antigen peptide EBNA3, or antigen peptide LMP2, each synthesized based on its corresponding amino acid sequence) were added to the mature DCs described above. The mixture was sealed with Parafilm and placed in a MACSmix rotator, and rotated in a cell incubator for 4 h to facilitate peptide loading. After incubation, the EBV-antigen peptide-loaded DCs were irradiated at a dose of 40 Gy using an irradiator. Following irradiation, the EBV-antigen peptide-loaded DCs were collected by centrifugation into a 15 mL centrifuge tube.
(2) PBMCs were isolated from healthy donor blood by Lymphoprep density gradient centrifugation. CD8+ T cells were isolated from PBMCs using the EasySep^{™} Human CD8 Positive Selection Kit. The EBV-antigen peptide-loaded DCs and CD8+ T cells were added to a 12-well plate at a DC:T ratio of 1:2.5. IL-21 was added to a final concentration of 30 ng/mL, together with EBV antigen peptides at 5 µg/mL.
(3) The 12-well plate was incubated overnight at 37 °C in a 5% CO₂ incubator. On Day 1, 10 ng/mL IL-2, IL-7, and IL-15 were added to each well, followed by further culture at 37 °C in a 5% CO₂ incubator for 10 days. During culture, half of the medium was replaced every 3 days.
(4) On Day 14, irradiated EBV-antigen peptide-loaded DCs were prepared and used to perform a second round of *in vitro* stimulation with the cells from each culture system, and stimulation was continued for another 10 days.
(5) On Day 25, cells from each well were collected, washed twice with FACS buffer, and stained with CD8a-APC and PE-HLA-A*0201-restricted EBV-peptide tetramer (MBL company). After mixing, the cells were incubated at 4 °C in the dark for 30 min, washed three times with FACS buffer, and then resuspended in 2 mL of FACS buffer containing 1× DAPI working solution. The proportion (%) of CD8+ /tetramer+ cells was then analyzed.

### 3. Sorting of EBV-antigen-specific T cells and single-cell TCR sequencing

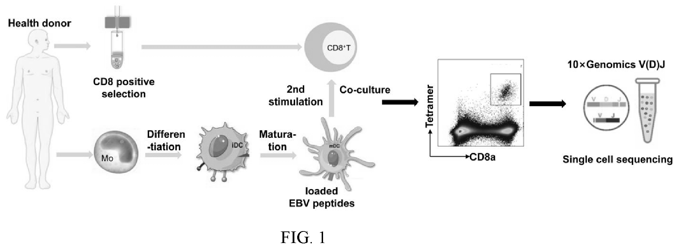

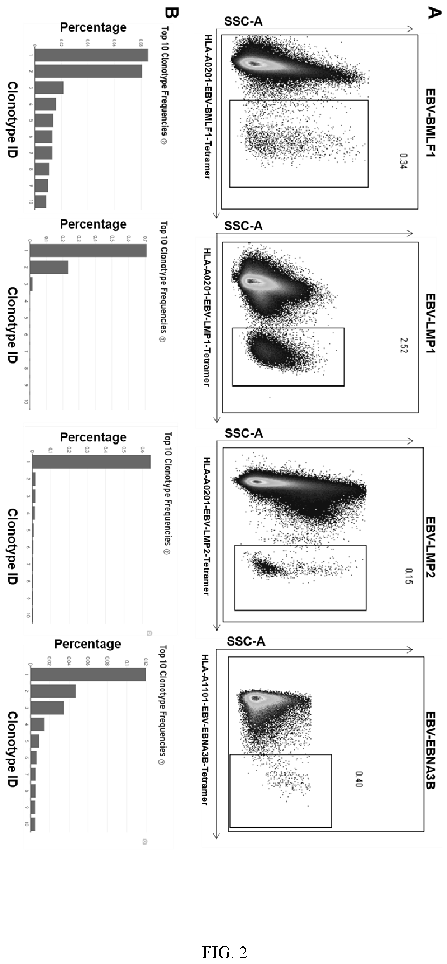

(1) The workflow for sorting EBV-antigen-specific T cells is shown in FIG. 1. CD8+ T cells from healthy donors were subjected to two rounds of *in vitro* stimulation with autologous EBV-antigen peptide-loaded DCs. Cells from the co-stimulatory culture system were then collected for flow cytometric analysis. Representative flow cytometry results are shown in FIG. 2A. The proportions of CD8a +/tetramer+ T cells specific for BMLF1, LMP1, EBNA3B, and LMP2 were 0.34%, 2.52%, 0.4%, and 0.15%, respectively, with clear population separation. These results indicate that EBV-antigen-specific TCR-T cells were specifically expanded during the two rounds of *in vitro* stimulation. Subsequently, CD8+/tetramer+ T cells were specifically sorted from the co-culture system using a flow cytometric cell sorter, and then sent to Genenergy Bio for 10× Genomics single-cell TCR and transcriptome sequencing, in order to obtain the TCR sequences and transcriptomic characteristics of CD8+/tetramer+ T cells.
(2) After completion of single-cell sequencing, transcriptomic characteristics were analyzed for the top ten ranked TCRs. The TCR sequencing results and frequency rankings of this cell population are shown in FIG. 2B. The TCR sequence with the highest frequency was then optimized by replacing its TCR constant region (TCR-C) with mTCR-C (wherein mTCR-C includes mTCRα-C and mTCRβ-C; the amino acid sequence of mTCRα-C is as shown in SEQ ID NO. 55, and the amino acid sequence of mTCRβ-C is as shown in SEQ ID NO. 56), so as to avoid TCR mispairing. The amino acid sequence of the TCR α-chain of the obtained T cell receptor BMLF1-TCR is as shown in SEQ ID NO. 8, and the amino acid sequence of the TCR β-chain of the obtained T cell receptor BMLF1-TCR is as shown in SEQ ID NO. 4.

The amino acid sequence of the TCR α-chain of the T cell receptor LMP1-TCR1 is as shown in SEQ ID NO. 16, and the amino acid sequence of the TCR β-chain of the T cell receptor LMP1-TCR1 is as shown in SEQ ID NO. 12.

The amino acid sequence of the TCR α-chain of the T cell receptor LMP1-TCR2 is as shown in SEQ ID NO. 24, and the amino acid sequence of the TCR β-chain of the T cell receptor LMP1-TCR2 is as shown in SEQ ID NO. 20.

The amino acid sequence of the TCR α-chain of the T cell receptor EBNA-3B-TCR is as shown in SEQ ID NO. 34, and the amino acid sequence of the TCR β-chain of the T cell receptor EBNA-3B-TCR is as shown in SEQ ID NO. 30.

The amino acid sequence of the TCR α-chain of the T cell receptor LMP2-TCR is as shown in SEQ ID NO. 43, and the amino acid sequence of the TCR β-chain of the T cell receptor LMP2-TCR is as shown in SEQ ID NO. 39.

For the T cell receptor BMLF1-TCR, the amino acid sequences of CDR α1, CDR α2, and CDR α3 in the TCR α-chain are as shown in SEQ ID NOs. 5, 6, and 7, respectively; and the amino acid sequences of CDR β1, CDR β2, and CDR β3 in the TCR β-chain are as shown in SEQ ID NOs. 1, 2, and 3, respectively.

For the T cell receptor LMP1-TCR1, the amino acid sequences of CDR α1, CDR α2, and CDR α3 in the TCR α-chain are as shown in SEQ ID NOs. 13, 14, and 15, respectively; and the amino acid sequences of CDR β1, CDR β2, and CDR β3 in the TCR β-chain are as shown in SEQ ID NOs. 9, 10, and 11, respectively.

For the T cell receptor LMP1-TCR2, the amino acid sequences of CDR α1, CDR α2, and CDR α3 in the TCR α-chain are as shown in SEQ ID NOs. 21, 22, and 23, respectively; and the amino acid sequences of CDR β1, CDR β2, and CDR β3 in the TCR β-chain are as shown in SEQ ID NOs. 17, 18, and 19, respectively.

For the T cell receptor EBNA-3B-TCR, the amino acid sequences of CDR α1, CDR α2, and CDR α3 in the TCR α-chain are as shown in SEQ ID NOs. 31, 32, and 33, respectively; and the amino acid sequences of CDR β1, CDR β2, and CDR β3 in the TCR β-chain are as shown in SEQ ID NOs. 27, 28, and 29, respectively.

For the T cell receptor LMP2-TCR, the amino acid sequences of CDR α1 CDR α2, and CDR α3 in the TCR α-chain are as shown in SEQ ID NOs. 40, 41, and 42, respectively; and the amino acid sequences of CDR β1, CDR β2, and CDR β3 in the TCR β-chain are as shown in SEQ ID NOs. 36, 37, and 38, respectively.

By using a P2A and Furin-cleavage (wherein the amino acid sequence of the P2A-Furin-cleavage fusion peptide is as shown in SEQ ID NO. 57), the TCR α-chain and β-chain can be co-expressed in a lentiviral expression vector. The nucleotide sequence encoding the T cell receptors that co-express the TCR α-chain and β-chain in the lentiviral expression vector is as shown in any one of SEQ ID NOs. 58-62. Specifically, the nucleotide sequence encoding the TCR α-chain and β-chain of the T cell receptor BMLF1-TCR is as shown in SEQ ID NO. 58; the nucleotide sequence encoding the TCR α-chain and β-chain of the T cell receptor LMP1-TCR1 is as shown in SEQ ID NO. 59; the nucleotide sequence encoding the TCR α-chain and β-chain of the T cell receptor LMP1-TCR2 is as shown in SEQ ID NO. 60; the nucleotide sequence encoding the TCR α-chain and β-chain of the T cell receptor EBNA-3B-TCR is as shown in SEQ ID NO. 61; and the nucleotide sequence encoding the TCR α-chain and β-chain of the T cell receptor LMP2-TCR is as shown in SEQ ID NO. 62. The above peptides and polynucleotides may be obtained by chemical synthesis or homologous recombination when the sequences are known.

### Example 2 In Vitro Binding Assay of EBV-Antigen-Specific TCR-T Cells

### 1. Preparation of EBV-antigen-specific TCR-T cells

(1) The optimized EBV-antigen-specific TCR obtained in Example 1 was constructed into a lentiviral expression vector, followed by lentiviral packaging of the EBV-antigen-specific TCR. The specific procedures for viral packaging were as follows: 293T cells were trypsinized and passaged, and cell density was observed on the day of transfection. Transfection was performed when the cells reached 80-90% confluency. No medium change was required prior to transfection. Opti-MEM was pre-warmed in a 37 °C water bath, and the Fugene-HD transfection reagent was equilibrated to room temperature and mixed well before use. The components of the transfection complex per T75 flask were as follows: pHR-SFFV-TCR-Puro plasmid containing the target TCR: 10 µg; pMD2.G (envelope plasmid): 5 µg; and pSPAX2 (packaging plasmid): 7.5 µg. Opti-MEM was added to a final volume of 1 mL and mixed thoroughly, followed by addition of 56 µL Fugene-HD transfection reagent. The mixture was gently pipetted 5-6 times and incubated at room temperature for 15 min, and then added to the T75 flask. Viral supernatants were collected at 48 h and 72 h post-transfection. After collection, the supernatants were centrifuged at 4000 rpm for 5 min and filtered through a 0.45 µm PES filter prior to use. EBV-antigen-specific TCR lentiviral stocks were collected at 48 h and 72 h after viral packaging.
(2) Jurkat T cells were infected with EBV-antigen-specific TCR lentiviral particles in the presence of 10 µg/mL polybrene, followed by centrifugation at 800×g for 90 min at 30 °C. After centrifugation, the cells were placed in a cell incubator and cultured at 37 °C in a 5% CO₂ incubator for 24 h, after which the medium was replaced with fresh RPMI-1640 medium for continued culture.
(3) CD8+ T cells from healthy donors were activated with 20 µL CD3/CD28 beads (Genscript) and 20 ng/mL IL-2. After 48 h of activation, 10 µg/mL polybrene was added, and a first round of infection with EBV-antigen-specific TCR lentivirus was performed. After centrifugation, the cells were cultured at 37 °C in a 5% CO₂ incubator for 24 h, followed by a second round of infection with EBV-antigen-specific TCR lentivirus.

### 2. FACS analysis of binding specificity of EBV-antigen-specific TCR-T cells

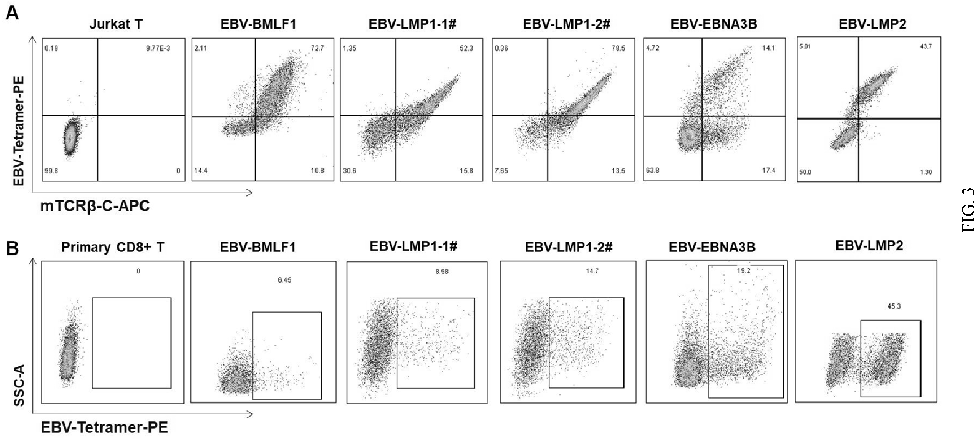

The prepared EBV-antigen-specific T cells described above were stained with mTCRβC+/tetramers. Flow cytometry results showed, for EBV-BMLF1-, LMP1-, and EBNA3B-specific TCRs, distinct tetramer+/mTCRβC+ Jurkat T cell populations (FIG. 3A) and distinct CD8+/tetramer+ populations among primary CD8+ T cells (FIG. 3B). These results demonstrate that the EBV-BMLF1/LMP1/EBNA3B-specific TCR screened and identified in the *in vitro* assay can be normally expressed in both Jurkat T cell lines and primary CD8+ T cells, and can specifically bind to HLA-restricted-EBV-tetramers.

### Example 3 In Vitro Activation Assay of EBV-Antigen-Specific TCR-T Cells

The *in vitro* activation assay of EBV-antigen-specific T cells, namely the IFNγ-Elispot assay, included the following steps:
(1) Autologous monocytes from donors were directionally differentiated into DCs *in vitro.* As described in Example 1, the DCs were respectively loaded with EBV antigen peptides, and the EBV-antigen-specific TCR-T cells prepared in Example 2 were co-cultured *in vitro* with the DCs loaded with EBV antigen peptides;
(2) After 24 h of *in vitro* co-culture, the cells were discarded, and the wells were washed five times with 200 µL DPBS to completely remove the cells. Subsequently, 100 µL of detection antibody 7-B6-1-biotin at a concentration of 1 µg/mL was added and incubated at 37 °C for 2 h. Thereafter, 100 µL streptavidin-HRP (3420-2H, MABTECH) was added and incubated at room temperature for 1 h;
(3) After washing five times with 200 µL DPBS, 100 µL of TMB substrate was added to each well until distinct spots appeared, and sterile water was then added to terminate the color development reaction.

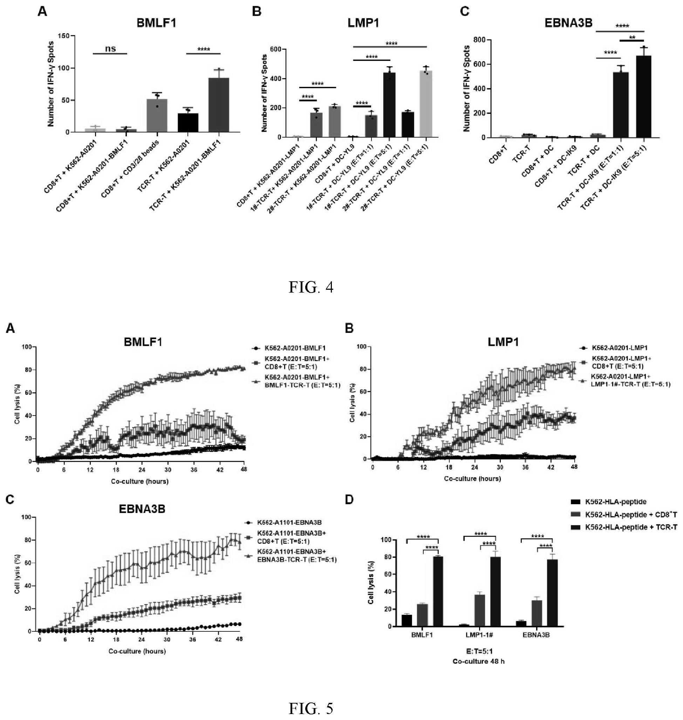

The results are shown in FIG. 4. DCs loaded with EBV antigen peptides were able to specifically activate EBV-antigen-specific TCR-T cells *in vitro.* After activation, the IFNγ expression level of EBV-antigen-specific TCR-T cells was significantly increased (FIGs. 4A-4C). These results indicate that the EBV-BMLF1/LMP1/EBNA3B-TCR screened and identified in the present study can specifically recognize EBV-BMLF1/LMP1/EBNA3B antigen peptides, respectively.

### Example 4 In Vitro Cytotoxicity Assay of EBV-Antigen-Specific TCR-T Cells

The *in vitro* cytotoxicity assay of EBV-TCR-T cells, namely the Incucyte killing assay, included the following steps:
(1) EBV-BMLF1/LMP1/EBNA3B-specific TCR-T effector cells and K562-A1101-BMLF1/LMP1/EBNA3B target cells were constructed *in vitro* by lentiviral infection of T cells and K562-A1101 cells, respectively. Subsequently, EBV-BMLF1/LMP1/EBNA3B-specific TCR-T cells or CD8+ T cells and K562-A1101 control cells or K562-A1101-BMLF1/LMP1/EBNA3B target cells were added to 96-well plates at an effector-to-target (E:T) ratio of 5:1 for co-culture;
(2) Incucyte^{®} Annexin V Dyes (Sartorius) were dissolved in 100 µL DPBS, and then diluted in complete culture medium to a final dilution of 1:200;
(3) The 96-well plates containing the cells were placed into an Incucyte (Sartorius) live-cell analysis system, and apoptosis was monitored using appropriate fluorescence channels. The specific parameters were as follows: A. objective magnification: 20×; B. channel selection: phase contrast, Green | Red | Orange | NIRc; C. scan type: 2-4 images per well; D. scan interval: once every 30 min, until completion of killing efficiency detection after 48 h of co-culture;
(4) After completion of detection, data analysis of the *in vitro* killing efficiency of TCR-T cells was performed using the Incucyte live-cell analysis system software (Sartorius).

The results are shown in FIG. 5. In this example, the Incucyte high-throughput long-term dynamic imaging and analysis system was used to monitor the killing efficiency of EBV-BMLF1/LMP1/EBNA3B-specific TCR-T cells against target cells in real time. The results demonstrated that, within 48 h of co-culture, compared with negative control CD8+ T cells not transduced with EBV-BMLF1/LMP1/EBNA3B-specific TCR, TCR-T cells at an effector-to-target (E:T) ratio of 5:1 were able to more efficiently kill target cells that were HLA-A*11:01-positive and expressed EBV-BMLF1/LMP1/EBNA3B antigens (FIGs. 5A-5D).

### Example 5 In Vivo Cytotoxicity Assay of EBV-Antigen-Specific TCR-T Cells

The *in vivo* cytotoxicity assay of EBV-EBNA3B-specific TCR-T cells, namely a CDX animal experiment, included the following steps:
(1) Six-week-old female NCG mice (purchased from GemPharmatech) were selected, and 2×10⁶ K562 cells carrying HLA-A*11:01 and the EBNA3B antigen and expressing a luciferase reporter gene were injected into the mice via the lateral tail vein;
(2) Three days after transplantation of the K562-HLA-EBNA3B-luci cell line, 1×10⁷ TCR-T cells were injected via the lateral tail vein, with T lymphocytes not transduced with a TCR gene serving as a negative control;
(3) On days 7, 15, and 27 after tumor cell inoculation, *in vivo* imaging of small animals was performed, and changes in body weight and survival of the mice were simultaneously monitored, so as to evaluate the *in vivo* cytotoxic effect of EBV-EBNA3B-specific TCR-T cells against target cells.

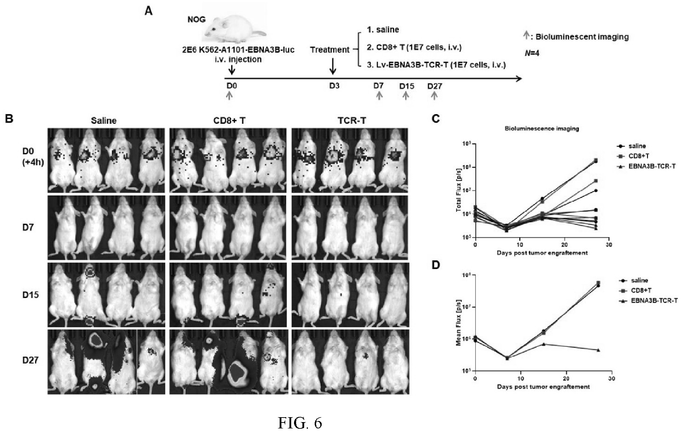

The results are shown in FIG. 6. In this example, the efficacy of EBV-EBNA3B-specific TCR-T cells in killing target cells was evaluated using an *in vivo* CDX model (FIG. 6A). *In vivo* imaging results showed that mice in the saline group and the mock T-cell group both exhibited obvious tumor cell growth within 27 days after tumor cell inoculation, whereas a portion of mice (3/4) receiving EBV-EBNA3B-specific TCR-T cells showed no obvious tumor growth (FIG. 6B). Moreover, the total photon flux or average photon intensity of luciferase signals in mice treated with EBV-EBNA3B-specific TCR-T cells was significantly lower than that in the saline group and the mock T-cell control group (FIGs. 6C-6D). The CDX experimental results demonstrate that HLA-A*11:01-restricted EBV-EBNA3B-specific TCR-T cells can efficiently and specifically kill K562-HLA-A*11:01-EBNA3B-luciferase target cells *in vivo.*

The above examples are provided to illustrate the embodiments disclosed herein and should not be construed as limiting the present disclosure. In addition, various modifications and changes to the methods described herein will be apparent to those skilled in the art without departing from the scope and spirit of the present disclosure. Although the present disclosure has been described in detail with reference to multiple specific preferred embodiments, it should be understood that the present disclosure is not limited to these specific embodiments. In fact, all such modifications that are obvious to those skilled in the art in light of the foregoing description are intended to be included within the scope of the present disclosure.

## Claims

1. A T cell receptor, comprising a T cell receptor BMLF1-TCR, a T cell receptor LMP1-TCR1, a T cell receptor LMP1-TCR2, a T cell receptor EBNA-3B-TCR, or a T cell receptor LMP2-TCR, wherein:
I) the T cell receptor BMLF1-TCR comprises a TCR α-chain variable region and a TCR β-chain variable region; wherein the TCR α-chain variable region of the T cell receptor BMLF1-TCR comprises CDR α1, CDR α2, and CDR α3, and the TCR β-chain variable region of the T cell receptor BMLF1-TCR comprises CDR β1, CDR β2, and CDR β3; wherein an amino acid sequence of CDR α3 of the T cell receptor BMLF1-TCR is as shown in SEQ ID NO. 7, and an amino acid sequence of CDR β3 of the T cell receptor BMLF1-TCR is as shown in SEQ ID NO. 3;
II) a TCR α-chain variable region of the T cell receptor LMP1-TCR1 comprises CDR α1, CDR α2, and CDR α3, and a TCR β-chain variable region of the T cell receptor LMP1-TCR1 comprises CDR β1, CDR β2, and CDR β3; wherein an amino acid sequence of CDR α3 of the T cell receptor LMP1-TCR1 is as shown in SEQ ID NO. 15, and an amino acid sequence of CDR β3 of the T cell receptor LMP1-TCR1 is as shown in SEQ ID NO. 11;
III) a TCR α-chain variable region of the T cell receptor LMP1-TCR2 comprises CDR α1, CDR α2, and CDR α3, and a TCR β-chain variable region of the T cell receptor LMP1-TCR2 comprises CDR β1, CDR β2, and CDR β3; wherein an amino acid sequence of CDR α3 of the T cell receptor LMP1-TCR2 is as shown in SEQ ID NO. 23, and an amino acid sequence of CDR β3 of the T cell receptor LMP1-TCR2 is as shown in SEQ ID NO. 19;
IV) a TCR α-chain variable region of the T cell receptor EBNA-3B-TCR comprises CDR α1, CDR α2, and CDR α3, and a TCR β-chain variable region of the T cell receptor EBNA-3B-TCR comprises CDR β1, CDR β2, and CDR β3; wherein an amino acid sequence of CDR α3 of the T cell receptor EBNA-3B-TCR is as shown in SEQ ID NO. 33, and an amino acid sequence of CDR β3 of the T cell receptor EBNA-3B-TCR is as shown in SEQ ID NO. 29;
V) a TCR α-chain variable region of the T cell receptor LMP2-TCR comprises CDR α1, CDR α2, and CDR α3, and a TCR β-chain variable region of the T cell receptor LMP2-TCR comprises CDR β1, CDR β2, and CDR β3; wherein an amino acid sequence of CDR α3 of the T cell receptor LMP2-TCR is as shown in SEQ ID NO. 42, and an amino acid sequence of CDR β3 of the T cell receptor LMP2-TCR is as shown in SEQ ID NO. 38.

2. The T cell receptor according to claim 1, wherein the T cell receptor further comprises any one of the following:
A) in the TCR α-chain variable region of the T cell receptor BMLF1-TCR, an amino acid sequence of CDR α1 is as shown in SEQ ID NO. 5, or an amino acid sequence of CDR α2 is as shown in SEQ ID NO. 6; and/or, in the TCR β-chain variable region of the T cell receptor BMLF1-TCR, an amino acid sequence of CDR β1 is as shown in SEQ ID NO. 1, or an amino acid sequence of CDR β2 is as shown in SEQ ID NO. 2;
B) in the TCR α-chain variable region of the T cell receptor LMP1-TCR1, an amino acid sequence of CDR α1 is as shown in SEQ ID NO. 13, or an amino acid sequence of CDR α2 is as shown in SEQ ID NO. 14; and/or, in the TCR β-chain variable region of the T cell receptor LMP1-TCR1, an amino acid sequence of CDR β1 is as shown in SEQ ID NO. 9, or an amino acid sequence of CDR β2 is as shown in SEQ ID NO. 10;
C) in the TCR α-chain variable region of the T cell receptor LMP1-TCR2, an amino acid sequence of CDR α1 is as shown in SEQ ID NO. 21, or an amino acid sequence of CDR α2 is as shown in SEQ ID NO. 22; and/or, in the TCR β-chain variable region of the T cell receptor LMP1-TCR2, an amino acid sequence of CDR β1 is as shown in SEQ ID NO. 17, or an amino acid sequence of CDR β2 is as shown in SEQ ID NO. 18;
D) in the TCR α-chain variable region of the T cell receptor EBNA-3B-TCR, an amino acid sequence of CDR α1 is as shown in SEQ ID NO. 31, or an amino acid sequence of CDR α2 is as shown in SEQ ID NO. 32; and/or, in the TCR β-chain variable region of the T cell receptor EBNA-3B-TCR, an amino acid sequence of CDR β1 is as shown in SEQ ID NO. 27, or an amino acid sequence of CDR β2 is as shown in SEQ ID NO. 28;
E) in the TCR α-chain variable region of the T cell receptor LMP2-TCR, an amino acid sequence of CDR α1 is as shown in SEQ ID NO. 40, or an amino acid sequence of CDR α2 is as shown in SEQ ID NO. 41; and/or, in the TCR β-chain variable region of the T cell receptor LMP2-TCR, an amino acid sequence of CDR β1 is as shown in SEQ ID NO. 36, or an amino acid sequence of CDR β2 is as shown in SEQ ID NO. 37.

3. The T cell receptor according to claim 1, wherein the T cell receptor further comprises any one of the following:
A1) an amino acid sequence of the TCR α-chain variable region of the T cell receptor BMLF1-TCR is as shown in SEQ ID NO. 45; and/or, an amino acid sequence of the TCR β-chain variable region of the T cell receptor BMLF1-TCR is as shown in SEQ ID NO. 46;
B1) an amino acid sequence of the TCR α-chain variable region of the T cell receptor LMP1-TCR1 is as shown in SEQ ID NO. 47; and/or, an amino acid sequence of the TCR β-chain variable region of the T cell receptor LMP1-TCR1 is as shown in SEQ ID NO. 48;
C1) an amino acid sequence of the TCR α-chain variable region of the T cell receptor LMP1-TCR2 is as shown in SEQ ID NO. 49; and/or, an amino acid sequence of the TCR β-chain variable region of the T cell receptor LMP1-TCR2 is as shown in SEQ ID NO. 50;
D1) an amino acid sequence of the TCR α-chain variable region of the T cell receptor EBNA-3B-TCR is as shown in SEQ ID NO. 51; and/or, an amino acid sequence of the TCR β-chain variable region of the T cell receptor EBNA-3B-TCR is as shown in SEQ ID NO. 52;
E1) an amino acid sequence of the TCR α-chain variable region of the T cell receptor LMP2-TCR is as shown in SEQ ID NO. 53; and/or, an amino acid sequence of the TCR β-chain variable region of the T cell receptor LMP2-TCR is as shown in SEQ ID NO. 54.

4. The T cell receptor according to claim 1, wherein the T cell receptor further comprises any one of the following:
A2) an amino acid sequence of a TCR α-chain of the T cell receptor BMLF1-TCR is as shown in SEQ ID NO. 8; and/or, an amino acid sequence of a TCR β-chain of the T cell receptor BMLF1-TCR is as shown in SEQ ID NO. 4;
B2) an amino acid sequence of a TCR α-chain of the T cell receptor LMP1-TCR1 is as shown in SEQ ID NO. 16; and/or, an amino acid sequence of a TCR β-chain of the T cell receptor LMP1-TCR1 is as shown in SEQ ID NO. 12;
C2) an amino acid sequence of a TCR α-chain of the T cell receptor LMP1-TCR2 is as shown in SEQ ID NO. 24; and/or, an amino acid sequence of a TCR β-chain of the T cell receptor LMP1-TCR2 is as shown in SEQ ID NO. 20;
D2) an amino acid sequence of a TCR α-chain of the T cell receptor EBNA-3B-TCR is as shown in SEQ ID NO. 34; and/or, an amino acid sequence of a TCR β-chain of the T cell receptor EBNA-3B-TCR is as shown in SEQ ID NO. 30;
E2) an amino acid sequence of a TCR α-chain of the T cell receptor LMP2-TCR is as shown in SEQ ID NO. 43; and/or, an amino acid sequence of a TCR β-chain of the T cell receptor LMP2-TCR is as shown in SEQ ID NO. 39.

5. An EBV antigen peptide, comprising an antigen peptide BMLF1, an antigen peptide LMP1, an antigen peptide EBNA3, or an antigen peptide LMP2;
wherein an amino acid sequence of the antigen peptide BMLF1 is as shown in SEQ ID NO. 25; or an amino acid sequence of the antigen peptide LMP1 is as shown in SEQ ID NO. 26; or an amino acid sequence of the antigen peptide EBNA3 is as shown in SEQ ID NO. 35; or an amino acid sequence of the antigen peptide LMP2 is as shown in SEQ ID NO. 44.

6. A biological material, comprising any one of the following:
a) a polynucleotide encoding the T cell receptor according to any one of claims 1-4; or a polynucleotide encoding the EBV antigen peptide according to claim 5;
b) a nucleic acid construct comprising the polynucleotide of a) and a plasmid backbone;
c) a virus comprising the polynucleotide of a);
d) a cell comprising the T cell receptor according to any one of claims 1-4, the EBV antigen peptide according to claim 5, the polynucleotide of a), the nucleic acid construct of b), or the virus of c).

7. The biological material according to claim 6, wherein the plasmid backbone of the nucleic acid construct is a lentiviral, adenoviral, or adeno-associated viral plasmid backbone; preferably, the plasmid backbone is one or more of pLKO.1-CMV-tGFP, pLKO.1-puro-CMV-tGFP, pLKO.1-CMV-Neo, pLKO.1-Neo, pLKO.1-Neo-CMV-tGFP, pLKO.1-puro-CMV-TagCFP, pLKO.1-puro-CMV-TagYFP, pLKO.1-puro-CMV-TagRFP, pLKO.1-puro-CMV-TagFP635, pLKO.1-puro-UbC-TurboGFP, pLKO.1-puro-UbC-TagFP635, pLKO-puro-IPTG-1xLacO, pLKO-puro-IPTG-3xLacO, pLP1, pLP2, pLP/VSV-G, pENTR/U6, pLenti6/BLOCK-iT-DEST, pcDNA1.2/V5-GW/lacZ, pLenti6.2/N-Lumio/V5-DEST, pGCSIL-GFP, and pHR-SFFV-Puro;
wherein the cell is a T cell; preferably, the T cell is a TCR-T cell; and/or, the T cell is a helper T cell, a suppressor T cell, an effector T cell, a cytotoxic T cell, a delayed-type hypersensitivity T cell, a natural T cell, or a memory T cell; preferably, the T cell is a Jurkat T cell or a CD8+ T cell.

8. Use of the T cell receptor according to any one of claims 1-4, the EBV antigen peptide according to claim 5, or the biological material according to any one of claims 6-7 in the preparation of a tumor therapeutic product, an anti-tumor peptide vaccine product, or a product for generating the T cell receptor *in vitro.*

9. The use according to claim 8, wherein the tumor comprises one or more of adrenocortical carcinoma, bladder urothelial carcinoma, breast cancer, cervical squamous cell carcinoma, cervical adenocarcinoma, cholangiocarcinoma, colon adenocarcinoma, lymphoid tumors, esophageal cancer, glioblastoma multiforme, head and neck squamous cell carcinoma, chromophobe renal cell carcinoma, clear cell renal cell carcinoma, papillary renal cell carcinoma, acute myeloid leukemia, low-grade glioma, hepatocellular carcinoma, mesothelioma, ovarian cancer, pancreatic cancer, pheochromocytoma, paraganglioma, prostate cancer, rectal cancer, malignant sarcoma, melanoma, gastric cancer, testicular germ cell tumor, thyroid cancer, thymic carcinoma, endometrial cancer, chronic myeloid leukemia, lung cancer, anal cancer, and retinoblastoma.

10. A tumor therapeutic product, comprising the T cell receptor according to any one of claims 1-4, the EBV antigen peptide according to claim 5, or the biological material according to any one of claims 6-7, and a pharmaceutically acceptable auxiliary material.

11. A method for treating a tumor, comprising administering an effective amount of the T cell receptor according to any one of claims 1-4, the EBV antigen peptide according to claim 5, the biological material according to any one of claims 6-7, or the tumor therapeutic product according to claim 10 to a subject in need thereof.

12. The method according to claim 11, wherein the method comprises one or more of the following:
1) the amount administered to the subject is 1-1000 mg/kg/day;
2) the subject is a mammal; preferably, the subject is a human.
